# EUROPÄISCHE PATENTSCHRIFT

(11) **EP 1 776 363 B1**
(45) Veröffentlichungstag und Bekanntmachung des Hinweises auf die Patenterteilung: **19.09.2012**
(21) Anmeldenummer: 05759648.8
(22) Anmeldetag: 12.07.2005
(51) Int. Cl.: C07D 475/00, A61K 31/519

(54) **NEUE 6-FORMYL-TETRAHYDROPTERIDINE, VERFAHREN ZU IHRER HERSTELLUNG UND IHRE VERWENDUNG ALS ARZNEIMITTEL U.A. GEGEN KREBS**
NOVEL 6-FORMYLTETRAHYDROPTERIDINES METHOD FOR PRODUCTION AND USE THEREOF AS MEDICAMENT AGAINST CANCER AMONGST OTHER THINGS
NOUVELLES 6-FORMYLTETRAHYDROPTERIDINES, PROCEDE DE PRODUCTION ET UTILISATION DE CES DERNIERES COMME MEDICAMENTS, NOTAMMENT CONTRE LE CANCER

(30) Priorität: 16.07.2004 DE 102004034623
(43) Veröffentlichungstag der Anmeldung: 25.04.2007
(73) Patentinhaber: BOEHRINGER INGELHEIM INTERNATIONAL GMBH, 55216 Ingelheim (DE); Boehringer Ingelheim Pharma GmbH & Co. KG, 55216 Ingelheim (DE)
(72) Erfinder: HOFFMANN, Matthias, 88441 Mittelbiberach (DE); GRAUERT, Matthias, 88400 Biberach (DE); STEEGMAIER, Martin, 72762 Reutlingen (DE); SOLCA, Flavio, A-1230 Wien (AT)
(74) Vertreter: Hammann, Heinz
(86) Internationale Anmeldenummer: PCT/EP2005/007532
(87) Internationale Veröffentlichungsnummer: WO 2006/008028

(56) Entgegenhaltungen:
- WO-A-01/19825
- US-A1- 2004 029 885

## Beschreibung

Die vorliegende Erfindung betrifft neue 6-Formyl-tetrahydropteridine der allgemeinen Formel (I) 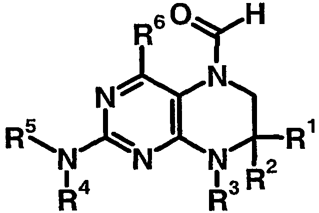 wobei die Reste R¹ bis R⁶ die in den Ansprüchen und der Beschreibung genannten Bedeutungen haben, deren Isomere, Verfahren zur Herstellung dieser 6-Formyl-tetrahydropteridine sowie deren Verwendung als Arzneimittel.

### Hintergrund der Erfindung

Pteridinon-Derivate sind als Wirkstoffe mit antiproliferativer Wirkung aus dem Stand der Technik bekannt. WO 01/019825 und WO 03/020722 beschreiben die Verwendung von Pteridinonderivaten zur Behandlung von Tumorerkrankungen.

Tumorzellen entziehen sich teilweise oder völlig der Regulation und Kontrolle durch den Organismus und zeichnen sich durch ein unkontrolliertes Wachstum aus. Dies beruht einerseits auf dem Verlust von Kontroll-Proteinen, wie z.B. Rb, p16, p21 und p53 als auch auf der Aktivierung von so genannten Beschleunigern des Zellzykluses, den cyclin-abhängigen Kinasen (CDK's).

Darüber hinaus wird auch für die Proteinkinase Aurora B eine essentielle Funktion beim Eintritt in die Mitose beschrieben. Aurora B phosphoryliert Histon H3 an Ser10 und leitet damit die Chromosomenkondensation ein (Hsu et al. 2000, Cell 102:279-91). Ein spezifischer Zellzyklusarrest in der G2/M Phase kann aber auch z.B. durch Inhibition von spezifischen Phosphatasen wie z.B. Cdc25C (Russell and Nurse 1986. Cell 45: 145-53) ausgelöst werden. Hefen mit defektem Cdc25 Gen arretieren in der G2 Phase, während eine Überexpression von Cdc25 zu einem verfrühten Eintritt in die Mitosephase führt (Russell und Nurse 1987, Cell 49:559-67). Desweiteren kann ein Arrest in der G2/M Phase auch durch Inhibition von bestimmten Motorproteinen, den so genannten Kinesinen wie z.B. Eg5 (Mayer et al. 1999, Science 286:971-4), oder durch Mikrotubuli stabilisierende oder destabilisierende Agentien (z.B. Colchicin, Taxol, Etoposid, Vinblastin, Vincristin) ausgelöst werden (Schiff und Horwitz 1980, Proc Natl Acad Sci U S A 77:1561-5).

Neben den Cyclin-abhängigen und den Aurora Kinasen spielen des weiteren die so genannten Polo-like Kinasen; eine kleine Familie von Serin/Threonin-Kinasen, eine wichtige Rolle bei der Regulation des eukaryontischen Zellzykluses. Bisher wurden die Polo-like Kinasen PLK-1, PLK-2, PLK-3 und PLK-4 in der Literatur beschrieben. Besonders für PLK-1 wurde eine zentrale Rolle in der Regulation der Mitosephase gezeigt. PLK-1 ist für die Reifung der Zentrosomen, für die Aktivierung der Phosphatase Cdc25C, sowie für die Aktivierung des Anaphase Promoting Complex verantwortlich (Glover et al. 1998, Genes Dev. 12:3777-87; Qian et al. 2001, Mol Biol Cell. 12:1791-9). Die Injektion von PLK-1 Antikörpern führt zu einem G2 Arrest in nicht transformierten Zellen, während Tumorzellen in der Mitosephase arretieren (Lane und Nigg 1996, J Cell Biol. 135:1701-13). Überexpression von PLK-1 konnte für verschiedene Tumorarten, wie nicht kleinzelliges Lungenkarzinom, Plattenepithelkarzinom, Brust- und kolorektales Karzinom (Wolf et al. 1997, Oncogene 14 :543 -549; Knecht etal. 1999, Cancer Res. 59:2794 -2797; Wolf et al. 2000, Pathol. Res. Pract. 196:753 -759; Takahashi et al. 2003, Cancer Sci. 94:148-52) gezeigt werden. Daher stellt diese Klasse von Proteinen ebenfalls einen interessanten Angriffspunkt zur therapeutischen Intervention proliferativer Krankheiten dar (Liu and Erikson 2003, Proc Natl Acad Sci U S A 100:5789-5794).

Die Resistenz vieler Tumorarten erfordert die Entwicklung neuer Arzneimittel zur Tumorbekämpfung.

Es ist die Aufgabe der vorliegenden Erfindung neue Verbindungen mit antiproliferativer Wirkung bereitzustellen.

### Detaillierte Beschreibung der Erfindung

Überraschenderweise wurde gefunden, dass Verbindungen der allgemeinen Formel (I), worin die Reste R¹ bis R⁶ die nachstehend genannten Bedeutungen haben, als Inhibitoren spezifischer Zellzykluskinasen, insbesondere der Polo-like Kinasen, wirken. Die genannten Verbindungen zeigen antiproliferative Wirkung, indem sie Zellen in der Mitosephase des Zellzykluses arretieren bevor der programmierte Zelltod in den arretierten Zellen eingeleitet wird. Somit können die erfindungsgemäßen Verbindungen beispielsweise zur Behandlung von Erkrankungen, die mit der Aktivität spezifischer Zellzykluskinasen in Zusammenhang stehen und durch exzessive oder anomale Zellproliferation charakterisiert sind, verwendet werden.

Die vorliegende Erfindung betrifft daher Verbindungen der allgemeinen Formel (I) 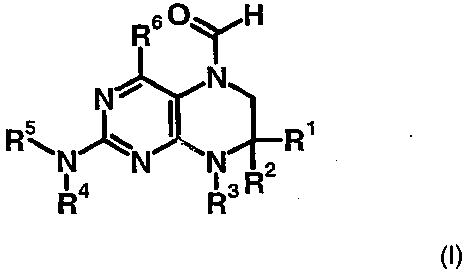 worin
R¹, R² gleich oder verschieden, ein Rest ausgewählt aus der Gruppe bestehend aus gegebenenfalls substituiertem C₁-C₁₀-Alkyl, C₂-C₁₀-Alkenyl, C₂-C₁₀-Alkinyl, Aryl, Heteroaryl, C₃-C₈-Cycloalkyl, C₃₋C₈-Heterocycloalkyl, -X-Aryl, -X-Heteroaryl, -X-Cycloalkyl, -X-Heterocycloalkyl, -NR⁷-Aryl, -NR⁷-Heteroaryl, -NR⁷-Cycloalkyl und -NR⁷-Heterocycloalkyl,
   oder
ein Rest ausgewählt aus der Gruppe bestehend aus Wasserstoff, Halogen, COXR⁷, CON(R⁷)₂, COR⁷ und XR⁷,
   oder
R¹ und R² gemeinsam eine 2- bis 5-gliedrige Alkylbrücke, die 1 bis 2 Heteroatome enthalten kann,
R³ Wasserstoff oder ein Rest ausgewählt aus der Gruppe bestehend aus gegebenenfalls substituiertem C₁-C₁₂-Alkyl, C₂-C₁₂-Alkenyl, C₂-C₁₂-Alkinyl, Aryl, Heteroaryl , -C₃-C₁₂-Cycloalkyl; C₃-C₁₂-Cycloalkenyl, C₇-C₁₂-Polycycloalkyl, C₇-C₁₂-Polycycloalkenyl und C₅-C₁₂-Spirocycloalkyl oder
R¹ und R³ oder R² und R³ gemeinsam eine gesättigte oder ungesättigte C₃-C₄-Alkylbrücke, die 1 bis 2 Heteroatome enthalten kann,
R⁴ gegebenenfalls substituiertes Aryl, Benzyl oder Heteroaryl,
R⁵ Wasserstoff, -CO-NH-C₁-C₄-Alkyl, -CO-C₁-C₄-Alkyl oder -CO-X-C₁-C₄-Alkyl,
R⁶ ein Rest ausgewählt aus der Gruppe bestehend aus Wasserstoff, NH₂, XH, Halogen und einer gegebenenfalls durch ein oder mehrere Halogenatome substituierten C₁-C₃-Alkyl-Gruppe,
R⁷ jeweils unabhängig voneinander, Wasserstoff oder ein Rest ausgewählt aus der Gruppe bestehend aus gegebenenfalls substituiertem C₁-C₄-Alkyl, C₂-C₄-Alkenyl, C₂-C₄-Alkinyl, Benzyl und Phenyl,
   und
X O oder S
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze, Solvate oder Hydrate, bedeuten.

Bevorzugt sind Verbindungen der Formel (I), worin
R¹ bis R⁴ und R⁷ die angegebene Bedeutung aufweisen und
R⁵ und R⁶ Wasserstoff
bedeuten.

Weiterhin bevorzugt sind Verbindungen der Formel (I), worin
R³ bis R⁷ die angegebene Bedeutung aufweisen und
R¹, R² gleich oder verschieden, Wasserstoff oder ein Rest, ausgewählt aus der Gruppe bestehend aus gegebenenfalls substituiertem C₁-C₆-Alkyl, C₂-C₆-Alkenyl, und C₂-C₆-Alkinyl,
oder
R¹ und R² gemeinsam eine 2- bis 5-gliedrige Alkylbrücke
bedeuten.

Weiterhin bevorzugt sind Verbindungen der Formel (I), worin
R¹,R² und R⁴ bis R⁷ die angegebene Bedeutung aufweisen,
und
R³ gleich Wasserstoff oder ein Rest, ausgewählt aus der Gruppe bestehend aus gegebenenfalls substituiertem C₁-C₁₂-Alkyl, C₂-C₁₂-Alkenyl, C₂-C₁₂-Alkinyl und C₆-C₁₄-Aryl, oder
ein Rest ausgewählt aus der Gruppe bestehend aus gegebenenfalls substituiertem C₃-C₁₂-Cycloalkyl, C₃-C₁₂-Cycloalkenyl, C₇-C₁₂-Polycycloalkyl, C₇-C₁₂-Polycycloalkenyl und C₅-C₁₂-Spirocycloalkyl,
bedeutet.

Besonders bevorzugt sind Verbindungen der Formel (I), worin
R¹ bis R³ und R⁵ bis R⁷ die angegebene Bedeutung aufweisen,
und
R⁴ ein Rest der allgemeinen Formel 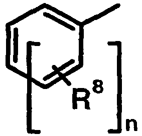 R⁸ gleich oder verschieden, Wasserstoff oder ein Rest ausgewählt aus der Gruppe bestehend aus gegebenenfalls substituiertem C₁-C₆-Alkyl, C₂-C₆-Alkenyl, C₂-C₆-Alkinyl, -O-C₁-C₆-Alkyl, -O-C₂-C₆-Alkenyl, -O-C₂-C₆-Alkinyl, Heterocycloalkyl, C₃-C₆-Cycloalkyl, Aryl, Heteroaryl, -O-Aryl, -O-Heteroaryl), -O-Cycloalkyl, und -O-Heterocycloalkyl oder
ein Rest ausgewählt aus der Gruppe bestehend aus Wasserstoff, -CONH₂, - COOR⁷, -OCON(R⁷)₂, -N(R⁷)₂, -NHCOR⁷, -NHCON(R⁷)₂, -NO₂, CF₃, Halogen, -O-C₁-C₆-Alkyl-Q¹, -CONR⁷-C₁-C₁₀-Alkyl-Q¹, -CONR⁷-C₁-C₁₀-Alkenyl-Q¹, -CONR⁷-Q², Halogen, OH, -SO₂R⁷, -SO₂N(R⁷)₂, -COR⁷, -COOR⁷, -N(R⁷)₂, -NHCOR⁷, - CONR⁷OC₁-C₁₀ -Alkyl-Q¹ und CONR⁷O-Q²,
oder
benachbarte Reste R⁸ gemeinsam eine Brücke der allgemeinen Formel a), b), c) oder d), 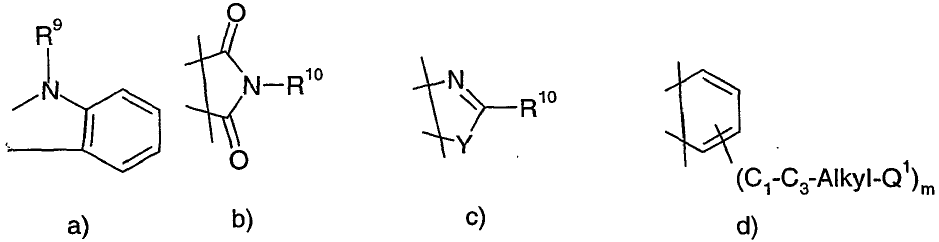 Y O, S oder NR¹¹,
m 0, 1 oder 2
R⁹ C₁-C₆-Alkyl
R¹⁰ Wasserstoff oder ein Rest ausgewählt aus der Gruppe bestehend aus gegebenenfalls substituiertem Phenyl, Pyridyl, Pyrazinyl, Pyrimidinyl, Pyridazinyl, -C₁-C₃-Alkyl-Phenyl, -C₁-C₃-Alkyl-Pyridyl, -C₁-C₃-Alkyl-Pyrazinyl, -C₁-C₃-Alkyl-Pyrimidinyl und -C₁-C₃-Alkyl-Pyridazinyl, Piperidinyl, Piperazinyl,
R¹¹ Wasserstoff oder C¹-C⁴-Alkyl
Q¹ Wasserstoff, -NHCOR⁷, oder ein Rest ausgewählt aus der Gruppe bestehend aus einer gegebenenfalls substituierten -NH-Aryl, -NH-Heteroaryl, Aryl- , Heteroaryl-, C₃-C₈-Cycloalkyl- und Heterocycloalkyl-Gruppe,
Q² Wasserstoff oder ein Rest ausgewählt aus der Gruppe bestehend aus einer gegebenenfalls substituierten Aryl-, Heteroaryl-, C₃-C₈-Heterocydoalkyl- ,C₃-C₈-Cycloalkyl- und C₁-C₄-Alkyl-C₃-C₈-cycloalkyl-Gruppe,
und
n 0, 1, 2, 3, 4 oder 5,
bedeuten.

Insbesondere bevorzugt sind Verbindungen der Formel (I), worin
Q¹, Q², n, R⁴ bis R⁸ die angegebene Bedeutung aufweisen,
R¹, R² gleich oder verschieden, Wasserstoff oder ein Rest ausgewählt aus der Gruppe bestehend Methyl, Ethyl, Propyl, Allyl und Propargyl
oder
R¹ und R² gemeinsam Cyclopropyl,
R³ gleich Wasserstoff, oder gegebenenfalls substituiertes C₁-C₆-Alkyl oder gegebenenfalls substituiertes C₃-C₁₂-Cycloalkyl,
bedeuten.

Überaus bevorzugt sind Verbindungen der Formel (I), worin
Q¹, Q², n, R¹ bis R⁴, R⁶ bis R⁷ die angegebene Bedeutung aufweisen,
und
R⁸ gleich oder verschieden, Wasserstoff oder einen Rest ausgewählt aus der Gruppe bestehend aus Halogen, (C₁-C₂-Alkyl)₂N-, CF₃, NH₂SO₂-, -CONH-C₆-C₁₄-Aryl, -CONH- C₁-C₄-alkyl-C₆-C₁₄-Aryl und -O-C₁-C₄-Alkyl, CONH-C₃-C₈-Cycloalkyl-heterocycloalkyl bedeuten.

Ein weiterer Gegenstand der Erfindung sind Verbindungen der Formel (I) zur Verwendung als Arzneimittel.

Erfindungsgemäß von besonderer Bedeutung sind Verbindung der Formel (I) zur Verwendung als Arzneimittel mit antiproliferativer Wirkung.

Ein weiterer Gegenstand der Erfindung ist die Verwendung einer Verbindung der Formel (I) zur Herstellung eines Arzneimittels zur Behandlung und/oder Prävention von Erkrankungen ausgewählt aus der Gruppe bestehend aus Krebs, bakteriellen und viralen Infektionen, Entzündungs- und Autoimmunerkrankungen, Chemotherapeutika induzierter Alopezie und Mukositis, kardiovaskulärer Erkrankungen, nephrologischen Erkrankungen, sowie chronisch und akut neurodegenerativen Erkrankungen, vorzugsweise zur Behandlung von Krebs, Entzündungs- und Autoimmunerkrankungen, insbesondere bevorzugt zur Behandlung von Krebs Und Entzündungserkranküngen.

Ein weiterer Gegenstand der Erfindung ist die Verwendung einer Verbindung der Formel (I) zur Herstellung eines Arzneimittels zur Inhibitierung der Polo-like Kinasen, insbesondere der Polo-like Kinase PLK-1.

Ein weiterer Gegenstand der Erfindung ist die Verwendung einer Verbindung der Formel (I) zur Herstellung eines Arzneimittels zur Behandlung und/oder Prävention von auf Überexpression der Polo-like Kinasen, insbesondere der PLK-1 Kinasen, beruhenden Tumorerkrankungen.

Ein weiterer Gegenstand der Erfindung sind pharmazeutische Zubereitungen, enthaltend als Wirkstoff eine oder mehrere Verbindungen der allgemeinen Formel (I) gegebenenfalls in Kombination mit üblichen Hilfs- und/oder Trägerstoffen.

Als Alkylgruppen sowie Alkylgruppen, welche Bestandteil anderer Reste sind, werden verzweigte und unverzweigte Alkylgruppen mit 1 bis 12 Kohlenstoffatomen bevorzugt 1 bis 6, besonders bevorzugt 1 bis 4 Kohlenstoffatomen bezeichnet, beispielsweise werden genannt: Methyl, Ethyl, Propyl, Butyl, Pentyl, Hexyl, Heptyl, Octyl, Nonyl, Decyl und Dodecyl. Sofern nicht anders genannt, sind von den vorstehend genannten Bezeichnungen Propyl, Butyl, Pentyl, Hexyl, Heptyl, Octyl, Nonyl, Decyl und Dodecyl sämtliche der möglichen isomeren Formen umfaßt. Beispielsweise umfaßt die Bezeichnung Propyl die beiden isomeren Reste n-Propyl und iso-Propyl, die Bezeichnung Butyl n-Butyl, iso-Butyl, sec. Butyl und tert.-Butyl, die Bezeichnung Pentyl, iso-Pentyl, Neopentyl etc.

In den vorstehend genannten Alkylgruppen können gegebenenfalls ein oder mehrere Wasserstoffatome durch andere Reste ersetzt sein. Beispielsweise können diese Alkylgruppen durch Methyl, Chlor oder Fluor, vorzugsweise Fluor, substituiert sein. Es können gegebenenfalls auch alle Wasserstoffatome der Alkylgruppe ersetzt sein.

Als Alkylbrücke werden, soweit nicht anders angegeben, verzweigte und unverzweigte Alkylgruppen mit 2 bis 5 Kohlenstoffatomen, beispielsweise Etyhlen, Propylen- , Isopropylen-, n-Butylen, iso-Butyl, sec. Butyl und tert.-Butyl etc. Brücken bezeichnet. Besonders bevorzugt sind Etyhlen, Propylen- und Butylen-Brücken. In den genannten Alkylbrücken können gegebenenfalls 1 bis 2 C-Atome durch ein oder mehrere Heteroatome ausgewählt aus der Gruppe Sauerstoff, Stickstoff oder Schwefel ersetzt sein.

Als Alkenylgruppen (auch soweit sie Bestandteil anderer Reste sind) werden verzweigte und unverzweigte Alkylengruppen mit 2 bis 10 Kohlenstoffatomen, bevorzugt 2 - 6 Kohlenstoffatomen, besonders bevorzugt 2 - 3 Kohlenstoffatomen betrachtet, soweit sie mindestens eine Doppelbindung aufweisen. Beispielsweise werden genannt: Ethenyl, Propenyl, Butenyl, Pentenyl etc. Sofern nicht anders genannt, sind von den vorstehend genannten Bezeichnungen Propenyl, Butenyl etc. sämtliche der möglichen isomeren Formen umfaßt. Beispielsweise umfaßt die Bezeichnung Butenyl 1-Butenyl, 2-Butenyl, 1-Methyl-1-Propenyl, 1-Methyl-2-Propenyl, 2-Methyl-1-Propenyl, 2-Methyl-2-Propenyl und 1-Ethyl-1-Ethenyl.

In den vorstehend genannten Alkenylgruppen können, soweit nicht anders beschrieben, gegebenenfalls ein oder mehrere Wasserstoffatome durch andere Reste ersetzt sein. Beispielsweise können diese Alkenylgruppen durch Methyl, Chlor oder Fluor, vorzugsweise Fluor, substituiert sein. Es können gegebenenfalls auch alle Wasserstoffatome der Alkenylgruppe ersetzt sein.

Als Alkinylgruppen (auch soweit sie Bestandteil anderer Reste sind) werden verzweigte und unverzweigte Alkinylgruppen mit 2 bis 10 Kohlenstoffatomen bezeichnet, soweit sie mindestens eine Dreifachbindung aufweisen, beispielsweise Ethinyl, Propargyl, Butinyl, Pentinyl, Hexinyl etc., vorzugsweise Ethinyl oder Propinyl.

In den vorstehend genannten Alkinylgruppen können, soweit nicht anders beschrieben, gegebenenfalls ein oder mehrere Wasserstoffatome durch andere Reste ersetzt sein. Beispielsweise können diese Alkinylgruppen durch Methyl, Chlor oder Fluor, vorzugsweise Fluor, substituiert sein. Es können gegebenenfalls auch alle Wasserstoffatome der Alkinylgruppe ersetzt sein.

Der Begriff Aryl steht für ein aromatisches Ringsystem mit 6 bis 14 Kohlenstoffatomen, vorzugsweise 6 oder 10 Kohlenstoffatomen, bevorzugt Phenyl, das, soweit nicht anders beschrieben, beispielsweise einen oder mehrere der nachfolgend genannten Substituenten tragen kann: OH, NO₂, CN, OMe, - OCHF₂, -OCF₃, -NH₂, Halogen, vorzugsweise Fluor oder Chlor, C₁-C₁₀-Alkyl, vorzugsweise C₁-C₅-Alkyl, bevorzugt C₁-C₃-Alkyl, besonders bevorzugt Methyl oder Ethyl, -O-C₁-C₃-Alkyl, vorzugsweise -O-Methyl oder -O-Ethyl, -COOH, -COO-C₁-C₄-Alkyl, vorzugsweise -O-Methyl oder -O-Ethyl, oder -CONH₂.

Als Heteroarylreste, in denen bis zu zwei C-Atome durch ein oder zwei Stickstoffatome ersetzt sind werden beispielsweise, Pyrrol, Pyrazol, Imidazol, Triazol, Pyridin, Pyrimidin, genannt, wobei jeder der vorstehend genannten Heteroarylringe gegebenenfalls ferner an einen Benzolring anneliert sein kann, vorzugsweise Benzimidazol, und wobei diese Heterocyclen ,soweit nicht anders beschrieben, beispielsweise einen oder mehrere der nachfolgend genannten Substituenten tragen können: F, Cl, Br, OH, OMe, Methyl, Ethyl, CN, CONH₂, NH₂, gegebenenfalls substituiertes Phenyl, gegebenenfalls substituiertes Heteroaryl, vorzugsweise gegebenenfalls substituiertes Pyridyl.

Als Cycloalkylreste werden Cycloalkylreste mit 3 - 12 Kohlenstoffatomen, vorzugsweise 3 - 8 Kohlenstoffatome, beispielsweise Cyclopropyl, Cyclobutyl, Cyclopentyl, Cyclohexyl, Cycloheptyl oder Cyclooctyl, vorzugsweise Cyclopropyl, Cyclopentyl oder Cyclohexyl bezeichnet, wobei jeder der vorstehend genannten Cycloalkylreste gegebenenfalls verbrückt und/oder ferner einen oder mehrere Substituenten tragen kann, beispielsweise: OH, NO₂, CN, OMe, -OCHF₂, -OCF₃, -NH₂ oder Halogen, vorzugsweise Fluor oder Chlor, C₁-C₁₀-Alkyl, vorzugsweise C₁-C₅-Alkyl, bevorzugt C₁-C₃-Alkyl, besonders bevorzugt Methyl oder Ethyl, -O-C₁-C₃-Alkyl, vorzugsweise -O-Methyl oder -O-Ethyl, -COOH, -COO-C₁-C₄-Alkyl, vorzugsweise -COO-Methyl oder -COO-Ethyl oder -CONH₂. Besonders bevorzugte Substituenten der Cycloalkylreste sind =O, OH, NH₂, Methyl oder F.

Als Cycloalkenylreste werden Cycloalkylreste mit 3 - 12 Kohlenstoffatomen, die mindestens eine Doppelbindung aufweisen, beispielsweise Cyclopropenyl, Cyclobutenyl, Cyclopentenyl, Cyclohexenyl oder Cycloheptenyl, vorzugsweise Cyclopropenyl, Cyclopententyl oder Cyclohexenyl bezeichnet, wobei jeder der vorstehend genannten Cycloalkenylreste gegebenenfalls verbrückt und/oder ferner einen oder mehrere Substituenten tragen kann.

"=O" bedeutet ein über eine Doppelbindung verknüpftes Sauerstoffatom.

Als Heterocycloalkylreste werden, soweit in den Definitionen nicht anders beschrieben, 3 bis 12 gliedrige, vorzugsweise 5- ,6- oder 7-gliedrige, gesättigte oder ungesättigte Heterocyclen, die als Heteroatome Stickstoff, Sauerstoff oder Schwefel enthalten können, beispielsweise Tetrahydrofuran, Tetrahydrofuranon, γ-Butylrolacton, α-Pyran, γ-Pyran, Dioxolan, Tetrahydropyran, Dioxan, Dihydrothiophen, Thiolan, Dithiolan, Pyrrolin, Pyrrolidin, Pyrazolin, Pyrazolidin, Imidazolin, Imidazolidin, Tetrazol, Piperidin, Pyridazin, Pyrimidin, Pyrazin, Piperazin, Triazin, Tetrazin, Morpholin, Thiomorpholin, Diazepan, Oxazin, Tetrahydro-oxazinyl, Isothiazol, Pyrazolidin genannt, vorzugsweise Morpholin, Pyrrolidin, Piperidin oder Piperazin, genannt, wobei der Heterocyclus gegebenenfalls verbrückt und/oder ferner Substituenten tragen kann, beispielsweise C1-C4-Alkyl, vorzugsweise.Methyl, Ethyl oder Propyl.

Als Polycycloalkylreste werden gegebenenfalls substituierte, bi-, tri-, tetra- oder pentacyclische Cycloalkylreste, beispielsweise Pinan, 2.2.2-Octan, 2.2.1-Heptan oder Adamantan, bezeichnet. Als Polycycloalkenylreste werden gegebenenfalls verbrückte oder/und substituierte, 8-gliedrige bi-, tri-, tetra- oder pentacyclische Cycloalkenyllreste, vorzugsweise Bicycloalkenyl- oder Tricycloalkenylreste, sofern sie mindestens eine Doppelbindung aufweisen, beispielsweise Norbornen, bezeichnet.

Als Spiroalkylreste werden gegebenenfalls substituierte spirocyclische C₅-C₁₂ Alkylreste bezeichnet.

Als Halogen wird im allgemeinen Fluor, Chlor, Brom oder Jod, vorzugsweise Fluor, Chlor oder Brom, besonders bevorzugt Chlor, bezeichnet.

Die erfindungsgemäßen Verbindungen können in Form der einzelnen optischen Isomeren, Mischungen der einzelnen Enantiomeren, Diastereomeren oder Racemate, in Form der Tautomere, in Form ihrer Solvate, vorzugsweise in Form ihrer Hydrate, sowie in Form der freien Basen oder der entsprechenden Säureadditionssalze mit pharmakologisch unbedenklichen Säuren - wie beispielsweise Säureadditionssalze mit Halogenwasserstoffsäuren, beispielsweise Chlor- oder Bromwasserstoffsäure, oder organische Säuren, wie beispielsweise Oxal-, Fumar-, Diglycol- oder Methansulfonsäure, vorliegen.

Der Substituent R¹ kann ein Rest ausgewählt aus der Gruppe bestehend aus gegebenenfalls substituiertem C₁-C₁₀-Alkyl, vorzugsweise C₁-C₄-Alkyl, besonders bevorzugt Methyl, Ethyl oder Propyl, C₂-C₁₀-Alkenyl, vorzugsweise Allyl, C₂-C₁₀-Alkinyl, vorzugsweise Propargyl, Aryl, vorzugsweise Phenyl, Heteroaryl, C₃-C₈-Cycloalkyl, C₃-C₈-Heterocycloalkyl, -X-Aryl, -X-Heteroaryl, -X-Cycloalkyl, -X-Heterocycloalkyl, -NR⁷-Aryl, -NR⁷-Heteroaryl, -NR⁷-Cycloalkyl und -NR⁷-Heterocycloalkyl,
oder
ein Rest ausgewählt aus der Gruppe bestehend aus Wasserstoff, Halogen, COXR⁷, CON(R⁷)₂, COR⁷ und XR⁷, bedeuten.

Vorzugsweise bedeutet der Substituent R¹ Ethyl oder Wasserstoff, besonders bevorzugt Wasserstoff.

Der Substituent R² kann ein Rest ausgewählt aus der Gruppe bestehend aus gegebenenfalls substituiertem C₁-C₁₀-Alkyl, vorzugsweise C₁-C₄-Alkyl, besonders bevorzugt Methyl, Ethyl oder Propyl, C₂-C₁₀-Alkenyl, vorzugsweise Allyl, C₂-C₁₀-Alkinyl, vorzugsweise Propargyl, Aryl, vorzugsweise Phenyl, Heteroaryl, C₃-C₈-Cycloalkyl, C₃-C₈-Heterocycloalkyl, -X-Aryl, -X-Heteroaryl, -X-Cycloalkyl, -X-Heterocycloalkyl, -NR⁷-Aryl, -NR⁷-Heteroaryl, -NR⁷-Cycloalkyl und -NR⁷-Heterocycloalkyl,
oder
ein Rest ausgewählt aus der Gruppe bestehend aus Wasserstoff, Halogen, COXR⁷, CON(R⁷)₂, COR⁷ und XR⁷, bedeuten
Vorzugsweise bedeutet der Substituent R2 Methyl, Ethyl, Allyl, Propargyl oder Wasserstoff, besonders bevorzugt Methyl oder Ethyl.

Die Substituenten R¹ und R² können gemeinsam eine 2- bis 5-gliedrige Alkylbrücke, vorzugsweise eine 2-gliedrige Alkylbrücke, die 1 bis 2 Heteroatome, beispielsweise Sauerstoff , Schwefel oder Stickstoff, vorzugsweise Sauerstoff oder Stickstoff, enthalten kann, bedeuten.

Der Substituent R³ kann Wasserstoff oder ein Rest ausgewählt aus der Gruppe bestehend aus gegebenenfalls substituiertem C₁-C₁₂-Alkyl, vorzugsweise C₂-C₆-Alkyl, besonders bevorzugt Pentyl, C₂-C₁₂-Alkenyl, C₂-C₁₂-Alkinyl, Aryl, Heteroaryl, C₃-C₁₂-Cycloalkyl, C₃-C₁₂-Cycloalkenyl, C₇-C₁₂-Polycycloalkyl, C₇-C₁₂-Polycycloalkenyl und C₅-C₁₂-Spirocycloalkyl oder
R¹ und R³ oder R² und R³ gemeinsam eine gesättigte oder ungesättigte C₃-C₄-Alkylbrücke, die 1 bis 2 Heteroatome enthalten kann, bedeuten. Vorzugsweise bedeutet der Substituent R₃ C₁-C₆-Alkyl oder -C₃-C₁₂-Cycloalkyl, insbesondere bevorzugt Pentyl oder Cyclopentyl.

Der Substituent R⁴ kann gegebenenfalls substituiertes Aryl, Benzyl oder Heteroaryl, vorzugsweise ein Rest der allgemeinen Formel 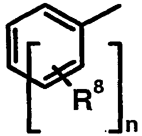 bedeuten.

Der Index n kann 0, 1, 2, 3, 4 oder 5, vorzugsweise 1 oder 2, besonders bevorzugt 1 bedeuten.

Der Substituent R⁵ kann ein Rest ausgewählt aus der Gruppe bestehend aus Wasserstoff, -CO-NH-C₁-C₄-Alkyl, -CO-C₁-C₄-Alkyl oder -CO-X-C₁-C₄-Alkyl, bedeuten. Vorzugsweise bedeutet der Substituent R⁵ Wasserstoff.

Der Substituent R⁶ kann ein Rest ausgewählt aus der Gruppe bestehend aus Wasserstoff, NH₂, XH, Halogen und einer gegebenenfalls durch ein oder mehrere Halogenatome substituierten C₁-C₃-Alkyl-Gruppe, bedeuten.

Vorzugsweise bedeutet der Substituent R⁶ Wasserstoff.

Der Substituent R⁷ kann jeweils unabhängig voneinander, Wasserstoff oder ein Rest ausgewählt aus der Gruppe bestehend aus gegebenenfalls substituiertem C₁-C₄-Alkyl, vorzugsweise Methyl oder Ethyl, C₂-C₄-Alkenyl, C₂-C₄-Alkinyl, Benzyl und Phenyl, bedeuten. Vorzugsweise bedeutet der Substituent R⁷ Wasserstoff.

X kann jeweils unabhängig voneinander, Sauerstoff oder Schwefel, vorzugsweise Sauerstoff, bedeuten.

Der Substituent R⁸ kann gleich oder verschieden, Wasserstoff oder ein Rest ausgewählt aus der Gruppe bestehend aus gegebenenfalls substituiertem C₁-C₆-Alkyl, C₂-C₆-Alkenyl, C₂-C₆-Alkinyl, -O-C₁-C₆-Alkyl, -O-C₂-C₆-Alkenyl, -O-C₂-C₆-Alkinyl, Heterocycloalkyl, C₃-C₆-Cycloalkyl, Aryl, Heteroaryl, -O-Aryl, -O-Heteroaryl, -O-Cycloalkyl, und -O-Heterocycloalkyl oder
ein Rest ausgewählt aus der Gruppe bestehend aus Wasserstoff, -CONH₂,-COOR⁷, -OCON(R⁷)₂, -N(R⁷)₂, -NHCOR⁷, -NHCON(R⁷)₂, -NO₂, CF₃, Halogen, -O-C₁-C₆-Alkyl-Q¹, -CONR⁷-C₁-C₁₀-Alkyl-Q¹, -CONR⁷-C₁-C₁₀-Alkenyl-Q¹, -CONR⁷-O², Halogen, OH, -SO₂R⁷, -SO₂N(R⁷)₂, -COR⁷, -COOR⁷, -N(R⁷)₂, -NHCOR⁷, - CONR⁷OC₁-C₁₀ -Alkyl-Q¹ und CONR⁷O-Q²,
oder
benachbarte Reste R⁸ gemeinsam eine Brücke der allgemeinen Formel a), b), c) oder d), 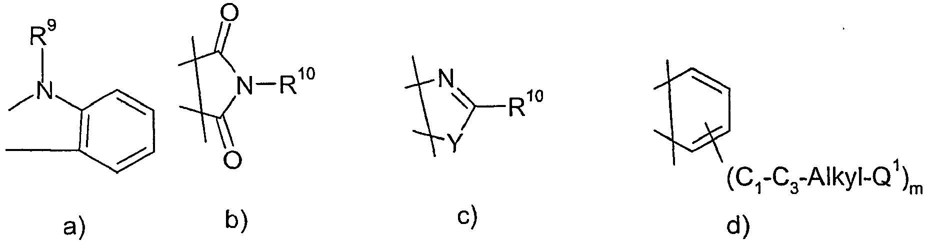 bedeuten.

Vorzugsweise bedeutet der Substituent R⁸ Aryl, bevorzugt Phenyl, Heteroaryl, besonders bevorzugt Pyridyl oder Pyrimidinyl, oder ein Rest aus der Gruppe , -CONR⁷-Q², bevorzugt -CONH-Q², -CONR⁷-C₁-C₁₀-Alkyl-Q¹, bevorzugt -CONH-C₁₋₂-Q, oder -CONH-C₂-Q¹, CONR⁷-C₃-C₈-Cycloalkyl-Q¹, bevorzugt -CONH-Cyclohexyl-Q¹ oder -CONH-Cyclopentyl-Q¹
Y kann O, S oder NR¹¹, vorzugsweise NR¹¹, bedeuten.
m 0, 1 oder 2, vorzugsweise 1, bedeuten.

Der Substituent R⁹ kann C₁-C₆-Alkyl, vorzugsweise Methyl, bedeuten.

Der Substituenten R¹⁰ kann Wasserstoff oder ein Rest ausgewählt aus der Gruppe bestehend aus gegebenenfalls substituiertem Phenyl, Pyridyl, Pyrazinyl, Pyrimidinyl, Pyridazinyl, Piperidinyl, Piperazinyl, -C₁-C₃-Alkyl-Phenyl, -C₁-C₃-Alkyl-Pyridyl, -C₁-C₃-Alkyl-Pyrazinyl, -C₁-C₃-Alkyl-Pyrimidinyl und -C₁-C₃-Alkyl-Pyridazinyl, bedeuten.

Besonders bevorzugt bedeutet R¹⁰ Pyridyl, Pyrimidinyl, Piperidinyl, Piperazinyl.

Der Substituent R¹¹ kann Wasserstoff oder C¹-C⁴-Alkyl, vorzugsweise Wasserstoff oder Methyl bedeuten.

Q¹ kann Wasserstoff, -NHCOR⁷, oder ein Rest ausgewählt aus der Gruppe bestehend aus einer gegebenenfalls substituierten -NH-Aryl, -NH-Heteroaryl, Aryl- , vorzugsweise Phenyl, Heteroaryl-, C₃-C₈-Cycloalkyl- und Heterocycloalkyl-Gruppe, bedeuten.

Besonders bevorzugt bedeutet Q¹ Heteroaryl oder Heterocycloalkyl, besonders bevorzugt, Pyridinyl, Pyrimidinyl, Morpholinyl, Piperazinyl oder Piperidinyl, Q²

Wasserstoff oder ein Rest ausgewählt aus der Gruppe bestehend aus einer gegebenenfalls substituierten Aryl-, vorzugsweise Phenyl, Heteroaryl-, C₃-C₈-Heterocycloalkyl- , C₃-C₈-Cycloalkyl- und C₁-C₄-Alkyl-C₃-C₈-cycloalkyl-Gruppe, bedeuten.

m kann 0, 1, 2, 3, 4 oder 5, vorzugsweise 1, bedeuten.

Die Verbindungen der allgemeinen Formel (I) können nach folgendem Syntheseverfahren hergestellt werden, wobei die Substituenten der allgemeinen Formeln (A1) bis (A4) und (I) die zuvor genannten Bedeutungen haben.

Dieses Verfahren ist als Erläuterung der Erfindung zu verstehen ohne selbige auf deren Gegenstand zu beschränken.

Eine Verbindung der Formel (A1) wird reduziert zur Verbindung der Formel (A2) welche anschließend mit Ameisensäure das 2-Chlor-6-formyl-tetrahydropteridin (A3) bildet. Dann werden Verbindungen der allgemeinen Formel (A3) mit einem substituierten Amin zur allgemeinen Formel (I) umgesetzt, welches gegebenenfalls weitere Transformationen erfahren kann. Verbindungen der Formel (A1) können gemäß WO 2003020722 erhalten werden. 4-Amino-*N*-cyclopropyl benzamid kann z.B. hergestellt werden nach folgender Literatur: B.W. Horrem und T.E. Lynes, J. Med. Chem. 1963, 6, 528-532. *trans*-4-Morpholino-cyclohexylamin **10** wurde durch folgende Vorschriften hergestellt:

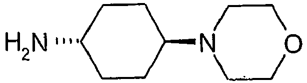

### Dibenzyl-4-morpholino-cyclohexylamin

3.9 g (30 mmol)) 4-Dibenzylcyclohexanon wurden in 100 mL CH₂Cl₂ gelöst und mit 3.9 g (45 mmol) Morpholin und 9,5 g (45 mmol) NaBH(OAc)₃ 12 Stunden bei 25°C gerührt. Anschließend wurde mit Wasser und Kaliumcarbonat versetzt, die organische Phase abgetrennt, getrocknet und eingeengt. Der Rückstand wurde über eine Kieselgelsäule gereinigt (Laufmittel: Essigester 90/ Methanol 10 + 1% konz. Ammoniak). Die geeigneten Fraktionen wurden im Vakuum eingeengt. Ausbeute: 6.6 g (60%) *cis*-Isomer und 2 g (18%) *trans*-Isomer.

Alternativ kann das *trans*-Dibenzyl-4-morpholino-cyclohexylamin nach folgendem Weg dargestellt werden:

33 g (112 mmol) 4-Dibenzylcyclohexanon wurden in 300 mL Methanol gelöst, mit 17.4 g (250 mmol) Hydroxylaminhydrochlorid versetzt und 4 Stunden bei 60°C gerührt. Das Lösungsmittel wurde im Vakuum eingeengt, mit 500 mL Wasser und 50 g Kaliumcarbonat versetzt und zwei Mal mit je 300 mL Dichlormethan extrahiert. Die organischen Phasen wurden getrocknet, im Vakuum eingeengt, der Rückstand aus Petrolether kristallisiert, in 1.5.L Ethanol gelöst und auf 70°C erwärmt. Es wurden 166 g Natrium portionsweise hinzugefügt und bis zur Auflösung des Natriums unter Rückfluss gekocht. Das Lösungsmittel wurde entfernt, der Rückstand mit 100 mL Wasser versetzt und zwei Mal mit je 400 mL Ether extrahiert. Die organischen Phasen wurden mit Wasser gewaschen, getrocknet, im Vakuum eingeengt und über eine Säule das *trans*-Isomer isoliert (Laufmittel: Essigester 80/ Methanol 20 + 2 % konz. Ammoniak).
Ausbeute: 12,6 g (41 %).

6.8 g (23 mmol) trans-1-Amino-4-dibenzylaminocyclohexan wurde in 90 mL DMF gelöst und mit 5 mL (42 mmol) 2,2'-Dichlorethylether und 5 g Kaliumcarbonat 8 Stunden bei 100°C gerührt. Nach Abkühlung wurde mit 30 mL Wasser versetzt, ausgefallene Kristalle abgesaugt und über eine kurze Säule (Laufmittel: Essigester) gereinigt. Der Rückstand wurde aus Methanol und konz. Salzsäure als Dihydrochlorid kristallisiert.
Ausbeute: 7.3 g (72%).

### Allgemeine Vorschriften:

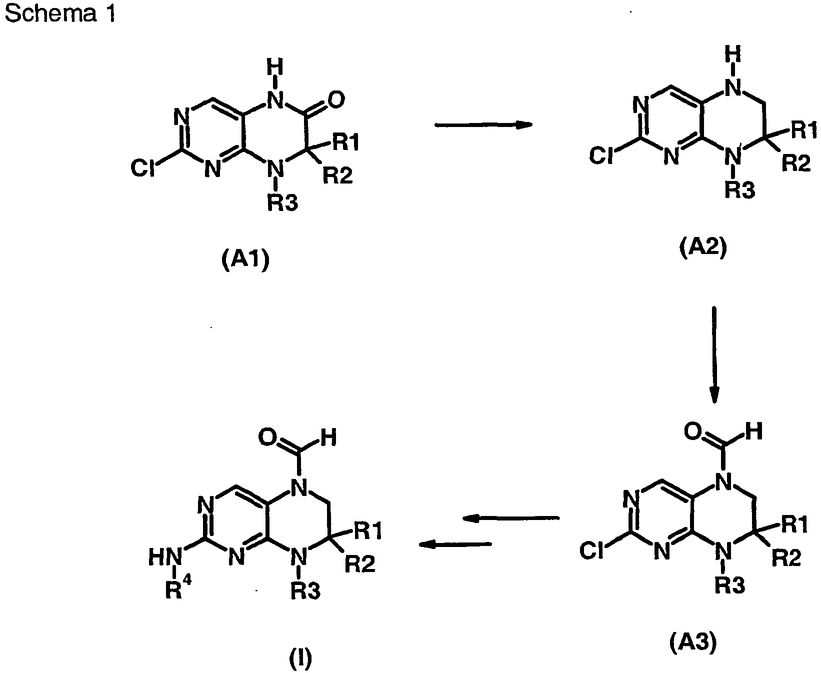

### Stufe 1

In Stufe 1 werden 1 Äquivalent der Verbindung (A1) und 1 bis 5 Äquivalente, bevorzugt 3-4 Äquivalente Natriumborhydrid mit Bortriflouridetherat in einem Verdünnungsmittel beispielsweise, Tetrahydrofuran, Diethylether oder Dioxan, vorzugsweise Tetrahydrofuran, bei 15-40°C für 12-24 h gerührt.

Zur Isolierung des Produktes wird anschließend die Reaktionsmischung mit Wasser und Salzsäure versetzt und das organische Lösungsmittel im Vakuum entfernt. Die wässrige Phase wird dann mit einer Base beispielsweise, Ammoniak oder Natriumcarbonat basisch gestellt und zwei bis dreimal mit einem organischen Lösungsmittels beispielsweise, Diethylether oder Ethylacetat, vorzugsweise Ethylacetat extrahiert. Die vereinigten organischen Extrakte werden getrocknet und das Lösungsmittel abdestilliert. Der Rückstand (Verbindung A2) kann ohne vorherige Aufreinigung in Stufe 2 eingesetzt werden.

### Stufe 2

Die in Stufe 1 erhaltene Verbindung (A2) wird in Ameisensäure gelöst und 5 Min bis 1 h, bevorzugt 15 Minuten unter Rückfluss zur Verbindung (A3) umgesetzt. Anschließend wird von der Ameisensäure abdestilliert und der Rückstand durch Zugabe eines oder mehrerer organischer Lösungsmittel beispielsweise Ethylacetat, Diethylether, Dichlormethan, Aceton, Petrolether rekristallisiert.

### Stufe 3

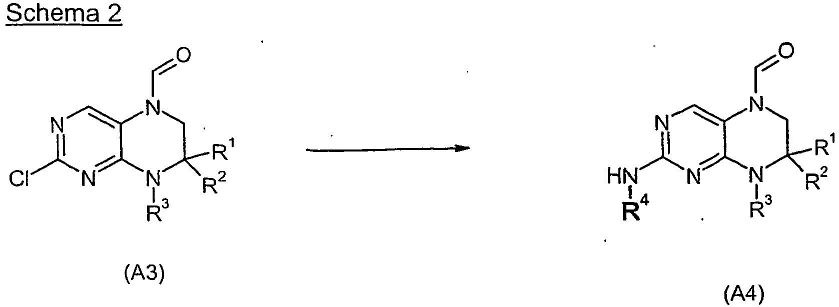

a) In Stufe 3 wird 1 Äquivalent der 2-Chlor-6-förmyl-tetrahydropteridines (A3) mit 1-3 Äquivalenten eines Amines vermischt und 30 Minuten bis 4 h, bei 120° bis 180°C, bevorzugt 160°C erhitzt (siehe Schema 2). Nach dem Abkühlen wird in einem geeigneten Lösungsmittel aufgenommen und das Produkt kristallisiert oder einer chromatographischen Reinigung unterworfen.
b) Alternativ kann in Stufe 3 auch 1 Äquivalent der 2-Chlor-6-formyl-tetrahydropteridins (A3) mit 1-3 Äquivalenten eines Amines in einem organischen Lösungsmittel beispielsweise Dioxan, Tetrahydrofuran mit 1 Äquivalent Säure, beispielsweise p-Toluolsulfonsäure 8 h bis 48h unter Rückfluss gerührt werden. Nach dem Abkühlen wird in einem geeigneten Lösungsmittel aufgenommen und das Produkt kristallisiert oder einer chromatographischen Reinigung unterworfen.

### Synthese der Beispiele 1 und 5 :

Zur Synthese der Beispiele 1 und 5 wird zunächst eine Zwischenverbindung **2** 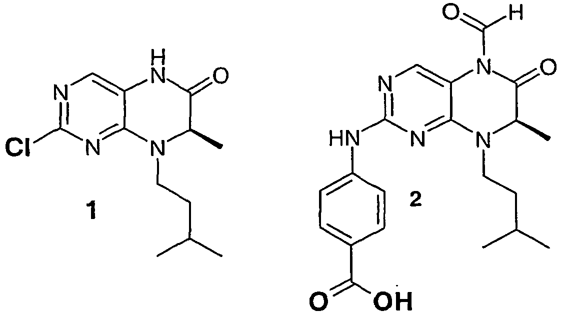 wie im folgenden beschrieben hergestellt.

### Synthese der Zwischenverbindung 2:

2 g der Verbindung **1** wird in 50 mL Tetrahydrofuran gelöst und mit 1 g Natriumborhydrid und 3 mL Bortrifluoridetherat bei 25°C 18 h gerührt. Dann wurden 2 mL Wasser und 20 mL 2N Salzsäure zugetropft und 10 Minuten unter Rückfluss erwärmt. Anschließend wurde vom Tetrahydrofuran abdestilliert, der Rückstand mit Ammoniaklösung versetzt und die wässrige Phase mit 2x jeweils 50 mL Ethylacetat extrahiert. Die organische Phase wurde mit Wasser gewaschen, getrocknet und im Vakuum eingeengt. Ausgefallene Kristalle wurden abfiltriert und mit Ether gewaschen. Es ergab 1,5 g einer Verbindung **3** die ohne weitere Reinigung für die nächste Umsetzung verwendet wurde.

1,4 g der Verbindung **3** wurden in 10 mL Ameisensäure gelöst und 15 Minuten unter Rückfluß gekocht. Dann wurde die Lösung im Vakuum eingeengt und der Rückstand mit Ether versetzt, der Niederschlag abfiltriert und mit Ether gewaschen. Dies ergab 1,2 g eines Produktes **4** das ohne weitere Reinigung in der nächsten Stufe eingesetzt wurde.

1,2 g der Verbindung **4** wurde mit 2,78 g 4-Aminobenzoesäureethylester ohne Lösungsmittel bei 150°C für 2h gerührt. Nach dem Abkühlen wurde der Reaktionsansatz mit 50 mL Ethylacetat versetzt, der entstandene Niederschlag abfiltriert und mit Ether gewaschen. Es ergab 1,1 g eines Produktes **5** das ohne weitere Reinigung für die nächste Stufe eingesetzt wurde.

1,1 g der Verbindung **5** wurde in 10 mL Methanol und 1 mL Wasser gelöst und mit 0,4 g Natriumhydroxid versetzt, dann wurde für 24 h bei 30°C gerührt. Anschließend wurde im Vakuum eingeengt, mit 20 mL Wasser und 0,8 mL Essigsäure versetzt und 2x mit je 50 mL Methylenchlorid extrahiert. Die organische Phase wurde getrocknet, im Vakuum eingeengt und aus Aceton kristallisiert. Es ergab 0,7 g eines Feststoffes **2** das für die weiteren Umsetzungen verwendet wurde.

Beispiel 1: 0,1 g **2** wurde zusammen mit 0,5 g Cyclopropylamin, 0,1 g o-Benzotriazolyl,N,N,N',N'-tetramethyluroniumtetrafluoroborat (TBTU), 0,5 mL N-Ethyldiisopropylamin in 2 mL Dimethylformamid für 30 Minuten gerührt. Dann wurde 50 mL Wasser, 1 g Kaliumcarbonat zugegeben und 2x mit je 50 mL Dichlormethan extrahiert. Die organische Phase wurde getrocknet und im Vakuum eingeengt. Anschließend wurde mittels Kieselgelchromatographie die Mischung fraktioniert, und das so erhaltene Rohprodukt in Aceton gelöst, die Lösung mit etherischer HCl versetzt, eingeengt und aus Ether kristallisiert. Es ergab 25 mg eines gelbes Pulvers.

Beispiel 5: 0,1g **2** wurde mit 0,15g 3-Aminopyridin, 0,1g TBTU, 0,5g N-Ethyldiisopropylamin in 2 mL Dimethylformamid 2 h bei 120°C gerührt. Anschließend wurde mit 50 mL Wasser, 1 g Kaliumcarbonat versetzt und 2x mit je 50 mL Methylenchlorid extrahiert. Die organische Phase wurde getrocknet, mittels Kieselgelchromatographie die Mischung fraktioniert, geeignete Fraktionen im Vakuum eingeengt und der Rückstand aus Aceton kristallisiert. Es ergab 10 mg eines gelben Feststoffes.

### Synthese der Beispiele 6 und 8 :

Zur Synthese der Beispiele 6 und 8 wird zunächst eine Zwischenverbindung **7** 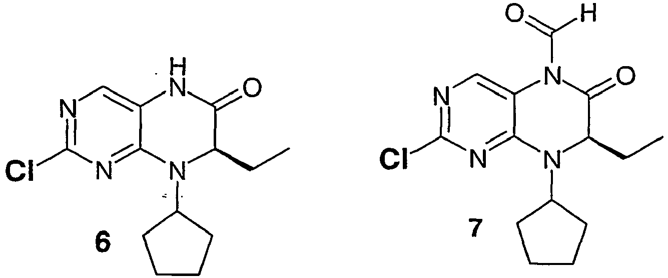 wie im folgenden beschrieben hergestellt.

### Synthese der Zwischenverbindung 7:

4 g der Verbindung **6** wird in 100 mL Tetrahydrofuran gelöst und mit 2 g Natriumborhydrid und 6 mL Bortrifluoridetherat bei 25°C 18 h gerührt. Dann wurde der Suspension langsam zuerst 4 mL Wasser dann 40 mL 2N Salzsäure zugetropft und die Mischung 10 Minuten unter Rückfluß erwärmt. Anschließend wurde vom Tetrahydrofuran abdestilliert, der Rückstand mit Ammoniaklösung versetzt und die wässrige Phase 2x mit jeweils 100 mL Ethylacetat extrahiert. Die organische Phase wurde mit Wasser gewaschen, getrocknet und im Vakuum eingeengt. Ausgefallene Kristalle wurden abfiltriert und mit Ether gewaschen. Es ergab 3 g einer Verbindung **8** die ohne weitere Reinigung für die nächste Umsetzung verwendet wurde.

0,6 g der Verbindung **8** wurden in 5 mL Ameisensäure gelöst und 15 Minuten unter Rückfluß erhitzt. Dann wurde die Lösung im Vakuum eingeengt und der Rückstand mit Ethylacetat und Petrolether versetzt, der Niederschlag abfiltriert und die Mutterlauge eingeengt. Dies ergab 0,58 g eines gelben öligen Produktes **7** das ohne weitere Reinigung für die Folgeumsetzungen verwendet wurde.

Beispiel 6: 0,1 g **7** wurde mit 0,14g 4-Amino-N-cyclopropylbenzamid ohne Lösungsmittel für 45 Minuten bei 160°C erhitzt. Nach dem Abkühlen wurde das Reaktionsgemisch in Dichlormethan und Methanol gelöst und über Kieselgel fraktioniert. Geeignete Fraktionen wurden vereinigt und im Vakuum eingeengt. Der Rückstand wurde in Ethylacetat gelöst mit Oxalatlösung aus Isopropanol, Diethylether und Petrolether versetzt und der entstandene Niederschlag abfiltriert und getrocknet. Es ergab 30 mg eines weißen Feststoffes.

Beispiel 8: 0,46 g **7** wurde mit 0,32 g 4-Amino-3-methoxybenzoesäure und 0,32 g p-Toluolsulfonsäure in 10 mL Dioxan unter Rückfluß für 48h gerührt. Die Reaktionsmischung wurde eingeengt und über Kieselgel fraktioniert. Geeignete Fraktionen wurden vereinigt und im Vakkum eingeengt. Der Rückstand wurde mit wenig Ethylacetat und Petrolether versetzt, der resultierende Niederschlag abfiltriert und getrocknet. Es ergab 0,3 g der Säure **9** als beiger Feststoff der für die weiteren Umsetzungen ohne zusätzliche Reinigung verwendet wurde.

0.065g **9** wurde zusammen mit 0,047 g TBTU, 0,2 mL Ethyldiisopropylamin in 2 mL Dichlormethan mit dem trans-4-Morpholino-cyclohexylamin **10** versetzt und 14 Stunden bei 25°C gerührt. Dann wurde mit weiterem Dichlormethan verdünnt und die organische Phase mit Wasser und Kaliumcarbonatlösung extrahiert. Anschließen wurde die organische Phase eingeengt und der Rückstand mittels Kieselgelchromatographie fraktioniert. Geeignete Fraktionen wurden eingeengt und der Rückstand durch Zugabe von Ethylacetat und Petrolether kristallisiert. Es ergab 50 mg eines weißen Feststoffes.

Analog zu den vorstehend beschriebenen Vorgehensweisen werden u.a. die in Tabelle 1 aufgeführten Verbindungen der allgemeinen Formel (I) erhalten.

**Tabelle 1**

| | | | | | | | | |
|---|---|---|---|---|---|---|---|---|
| 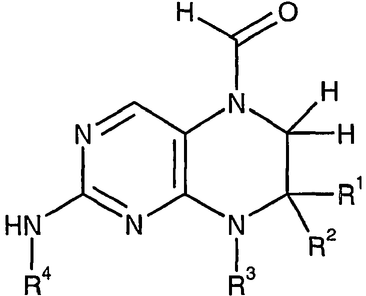 | | | | | | | | |

| Beispiel | Konfig. R1/R2 | R¹ | R² | R³ | R⁴ | Molekulargewicht | ESI, [M+H] | Schmelzpunkt |
|---|---|---|---|---|---|---|---|---|
| 1 | R | H | 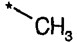 | 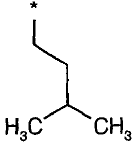 | 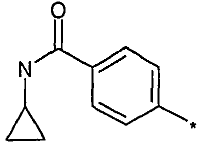 | 422,53 | 423 | |
| 2 | R | H | 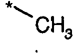 | 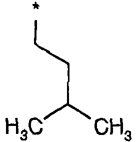 | 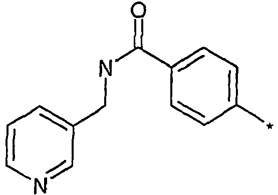 | 473,58 | 474 | |
| 3 | R | H | 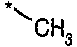 | 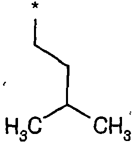 | 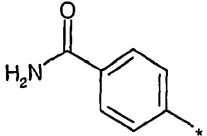 | 382,47 | 383 | |
| 4 | R | H | 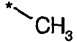 | 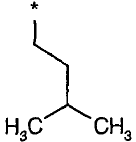 | 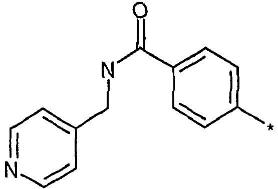 | 473,58 | 474 | |
| 5 | R | H | 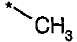 | 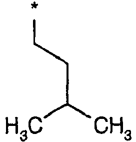 | 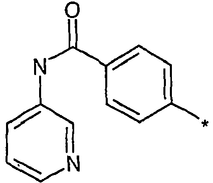 | 459,55 | 460 | |
| 6 | R | H | 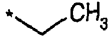 | 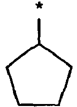 | 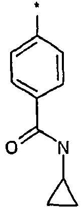 | 434,54 | 435 | |
| 7 | R | H | 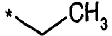 | 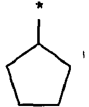 | 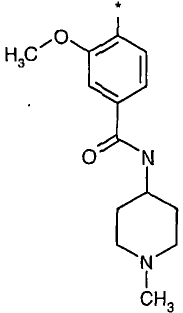 | 521,66 | 522 | |
| 8 | R | H | 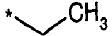 | 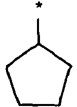 | 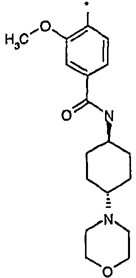 | 591,75 | 592 | 154°C |
| 9 | R | H | 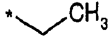 | 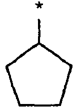 | 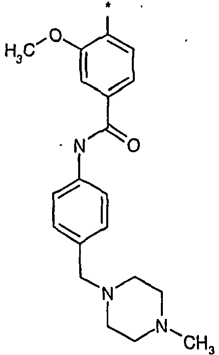 | 612,78 | 613 | 118°C |
| 10 | R | H | 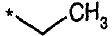 | 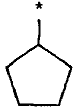 | 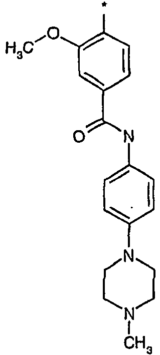 | 598,75 | 599 | 173°C |

| | | | | | | | | |
|---|---|---|---|---|---|---|---|---|
| *Bindungsstelle | | | | | | | | |

Wie gefunden wurde, zeichnen sich die Verbindungen der allgemeinen Formel (I) durch vielfältige Anwendungsmöglichkeiten auf therapeutischem Gebiet aus. Hervorzuheben sind solche Anwendungsmöglichkeiten, für welche die Inhibition spezifischer Zellzykluskinasen, insbesondere die inhibierende Wirkung auf die Proliferation kultivierter humaner Tumorzellen, aber auch auf die Proliferation anderer Zellen, wie z.B. Endothelzellen, eine Rolle spielen.

Wie durch DNA-Färbung mit darauf folgender FACS Analyse gezeigt werden konnte, ist die, durch die erfindungsgemäßen Verbindungen bewirkte, Proliferationsinhibition vermittelt durch einen Arrest der Zellen vor allem in der G2/M Phase des Zellzyklus. Die Zellen arretieren abhängig von den verwendeten Zellen für eine bestimmte Zeitspanne in dieser Zellzyklus Phase, bevor der programmierte Zelltod eingeleitet wird. Ein Arrest in der G2/M Phase des Zellzyklus wird z.B. durch die Inhibition spezifischer Zellzykluskinasen ausgelöst. Auf Grund ihrer biologischen Eigenschaften eignen sich die erfindungsgemäßen Verbindungen der allgemeinen Formel I, deren Isomere und deren physiologisch verträgliche Salze zur Behandlung von Erkrankungen, die durch exzessive oder anomale Zellproliferation charakterisiert sind.

Zu solchen Erkrankungen gehören beispielsweise: Virale Infektionen (z.B. HIV und Kaposi Sarkoma); Entzündung und Autoimmun-Erkrankungen (z.B. Colitis, Arthritis, Alzheimer Erkrankung, Glomerulonephritis und Wund-Heilung); bakterielle, fungale und/oder parasitäre Infektionen; Leukämien, Lymphoma und solide Tumore; Haut-Erkrankungen (z.B. Psoriasis); Knochen-Erkrankungen; kardiovaskuläre Erkrankungen (z.B. Restenose und Hypertrophie). Ferner sind sie nützlich als Schutz von proliferierenden Zellen (z.B. Haar-, Intestinal-, Blut - und Progenitor-Zellen) gegen DNA-Schädigung durch Strahlung, UV-Behandlung und/oder zytostatischer Behandlung (Davis et al., 2001).

Die neuen Verbindungen können zur Prävention, Kurz- oder Langzeitbehandlung der vorstehend genannten Erkrankungen, auch in Kombination mit anderen Wirkstoffen, die für dieselben Indikationen Verwendung finden, z.B. Cytostatika, Hormone oder Antikörper verwendet werden.

Die Wirkung der erfindungsgemäßen Verbindungen wurde im PLK1 Inhibitionsassay, im Cytotoxizitätstest an kultivierten humanen Tumorzellen und/oder in einer FACS-Analyse, beispielsweise an HeLa S3-Zellen, bestimmt. Die Verbindungen zeigten in beiden Testmethoden eine gute bis sehr gute Wirksamkeit, d.h. beispielsweise einen EC₅₀-Wert im HeLa S3-Cytotoxizitätstest kleiner 5 µmol/L, in der Reget kleiner 1 µmol/L und einen IC₅₀-Wert im PLK1-Inhibitionsassay kleiner 1 µmol/L.

### PLK1 Kinaseassay

### Enzymherstellung:

Rekombinantes humanes und an seinem N-terminalen Ende mit GST verbundenes PLK1 Enzym wird aus Bakulovirus infizierten Insektenzellen (Sf21) isoliert. Die Reinigung erfolgt durch Affinitätschromatographie an Glutathion Sepharose Säulen.

4x10⁷ Sf21 Zellen (Spodoptera frugiperda) in 200 ml Sf-900 II Serum freien Insektenzellmedium (Life Technologies) werden in eine Spinnerflasche ausgesät. Nach 72 Stunden Inkubation bei 27°C und 70 rpm werden 1x10⁸ Sf21 Zellen in insgesamt 180 ml Medium in eine neue Spinnerflasche ausgesät. Nach weiteren 24 Stunden werden 20 ml rekombinanter Baculovirus Stammsuspension zugesetzt und die Zellen 72 Stunden bei 27°C bei 70 rpm kultiviert. 3 Stunden vor dem Ernten wird Okadainsäure zugesetzt (Calbiochem, Endkonzentration 0,1 µM) und die Suspension weiter inkubiert. Die Zellzahl wird bestimmt, die Zellen abzentrifugiert (5 Minuten, 4°C, 800 rpm) und 1x mit PBS (8 g NaCl/l, 0,2 g KCl/l, 1,44 g Na₂HPO₄/l, 0,24 g KH₂PO4/l) gewaschen. Nach nochmaligem Abzentrifugieren wird das Pellet in flüssigem Stickstoff Schock gefroren. Danach wird das Pellet rasch aufgetaut und in eiskaltem Lysispuffer (50 mM HEPES pH 7,5, 10 mM MgCl₂, 1 mM DTT, 5 µg/ml Leupeptin, 5 µg/ml Aprotinin, 100 µM NaF, 100 µM PMSF, 10 mM ß-Glycerolphosphat, 0.1 mM Na₃VO₄, 30 mM 4-Nitrophenylphosphate) zu 1x10⁸ Zellen/ 17,5 ml resuspendiert. Die Zellen werden 30 Minuten auf Eis lysiert. Nach dem Entfernen der Zelltrümmer durch Zentrifugation (4000 rpm, 5 Minuten) wird der klare Überstand mit Glutathion Sepharosebeads versetzt (1 ml resuspendierte und gewaschene Beads für 50 ml Überstand) und 30 Minuten bei 4°C auf einem Rotationsbrett inkubiert. Danach werden die Beads mit Lysispuffer gewaschen und das rekombinante Protein mit 1 ml Elutionspuffer/ ml resuspendierte Beads (Elutionspuffer: 100 mM Tris/HCl pH=8,0, 120 mM NaCl, 20 mM reduziertes Glutathion (Sigma G-4251), 10 mM MgCl₂, 1 mM DTT) von den Beads eluiert. Die Proteinkonzentration wird mittels Bradford Assay bestimmt.

### Assaydurchführung:

In einem Napf einer 96-Loch Rundbodenplatte (Fa. Greiner bio-one, PS-Microtiterplatte Nr.650101) werden folgende Komponenten zusammengefügt:
- 10 µl zu testende Verbindung in variabler Konzentration (z.B. beginnend bei 300 µM, und Verdünnung in 1:3) in 6% DMSO, 0,5 mg/ml Casein (Sigma C-5890), 60 mM β-Glycerophosphat, 25 mM MOPS pH=7,0, 5 mM EGTA, 15 mM MgCl₂, 1 mM DTT
- 20 µl Substratlösung (25 mM MOPS pH=7,0, 15 mM MgCl₂, 1 mM DTT, 2,5 mM EGTA, 30 mM β-Glycerophosphat, 0,25 mg/ml Casein)
- 20 µl Enzymverdünnung (1:100 Verdünnung des Enzymstocks in 25 mM MOPS pH=7,0, 15 mM MgCl₂, 1 mM DTT)
- 10 µl ATP Lösung (45 µM ATP mit 1,11x10⁶ Bq/ml gamma-P33-ATP).

Durch Zusatz der ATP Lösung wird die Reaktion gestartet und 45 Minuten bei 30 °C unter leichtem Schütteln (650 rpm auf IKA Schüttler MTS2) durchgeführt. Die Reaktion wird durch Zusatz von 125 µl eiskalter 5%iger TCA pro Napf gestoppt und mindestens 30 Minuten auf Eis inkubiert. Das Präziptitat wird durch Ernten auf Filterplatten (96-well-Microtiter-Filterplatte: UniFilter-96, GF/B; Fa. Packard; Nr.6005177) übertragen, dann viermal mit 1%iger TCA gewaschen und bei 60°C getrocknet. Nach Zugabe von 35µl Szintillationslösung (Ready-Safe; Beckmann) pro Napf wird die Platte mit Sealing-tape zugeklebt und die präzipitierte Menge P33 mit dem Wallac Betacounter gemessen.

Die Messdaten werden mit der Standard Graphpad Software (Levenburg-Marquard Algorhythmus) ausgewertet.

### Messung der Cytotoxizität an kultivierten humanen Tumorzellen

Zur Messung der Cytotoxizität an kultivierten humanen Tumorzellen wurden Zellen der zervikalen Carcinoma Tumorzell-Linie HeLa S3 (erhalten von American Type Culture Collection (ATCC)) in Ham's F12 Medium (Life Technologies) und 10% fötalem Rinderserum (Life Technologies) kultiviert und in der log-Wachstumsphase geerntet. Anschließend wurden die HeLa S3 Zellen in 96-well Platten (Costar) mit einer Dichte von 1000 Zellen pro well eingebracht und über Nacht in einem Inkubator (bei 37°C und 5 % CO₂) inkubiert, wobei auf jeder Platte 6 wells nur mit Medium gefüllt wurden (3 wells zur Mediumkontrolle, 3 wells zur Inkubation mit reduzierten AlamarBlue Reagenz). Die Wirksubstanzen wurden in verschiedenen Konzentrationen (gelöst in DMSO; DMSO-Endkonzentration: 0.1%) zu den Zellen zugegeben (jeweils als Dreifachbestimmung). Nach 72 Stunden Inkubation wurden zu jeden well 20 µl AlamarBlue Reagenz(AccuMed International) zugesetzt, und die Zellen für weitere 7 Stunden inkubiert. Zur Kontrolle wurde zu 3 wells je 20 µl reduziertes AlamarBlue Reagenz gegeben (AlamarBlue Reagenz, das für 30 min autoklaviert wurde). Nach 7 h Inkubation wurde der Farbumsatz des AlamarBlue Reagenz in den einzelnen wells in einem Perkin Elmer Fluoreszenzspektrophotometer bestimmt (Exitation 530 nm, Emission 590 nm, Slits 15, Integrate time 0.1). Die Menge an umgesetzten AlamarBlue Reagenz repräsentiert die metabolische Aktivität der Zellen. Die relative Zellaktivität wurde in Prozent der Kontrolle (HeLa S3 Zellen ohne Inhibitor) berechnet und die Wirkstoffkonzentration, die die Zellaktivität zu 50% hemmt (IC₅₀) abgeleitet. Die Werte wurden hierbei aus dem Mittelwert von drei Einzelbestimmungen - unter Korrektur des Leerwertes (Mediumkontrolle)-berechnet.

### FACS- Analyse

Propidium Iodid (PI) bindet stöchiometrisch an doppelsträngige DNA, und ist damit geeignet den Anteil an Zellen in der G1, S, und G2/M Phase des Zellzykluses auf der Basis des zellulären DNA Gehaltes zu bestimmen. Zellen in der G0 und G1 Phase haben einen diploiden DNA Gehalt (2N), während Zellen in der G2 oder Mitose einen 4N DNA Gehalt haben.

Für eine PI-Färbung wurden beispielsweise 0.4 Mio HeLa S3 Zellen auf eine 75 cm² Zellkulturflasche ausgesät, nach 24 h wurde entweder 0.1 % DMSO als Kontrolle zugesetzt, bzw. die Substanz in verschiedenen Konzentrationen (in 0. 1 % DMSO). Die Zellen wurden für 24 h mit der Substanz bzw. mit DMSO inkubiert, bevor die Zellen 2 x mit PBS gewaschen und dann mit Trypsin /EDTA abgelöst wurden. Die Zellen wurden zentrifugiert (1000 Upm, 5 min, 4°C), und das Zellpellet 2 x mit PBS gewaschen, bevor die Zellen in 0.1 ml PBS resuspendiert wurden. Anschließend wurden die Zellen für 16 Stunden bei 4°C oder alternativ für 2 Stunden bei -20°C mit 80% Ethanol fixiert. Die fixierten Zellen (10⁶ Zellen) wurden zentrifugiert (1000 Upm, 5min, 4°C), mit PBS gewaschen und anschließend nochmals zentrifugiert. Das Zellpellet wurde in 2 ml Triton X-100 in 0.25 % PBS resuspendiert, und 5 min auf Eis inkubiert, bevor 5 ml PBS zugeben wurden und erneut zentrifugiert wurde. Das Zellpellet wurde in 350 µl PI Färbelösung (0.1 mg/ml RNase A, 10 µg/ml Prodium Iodid in 1 x PBS) resuspendiert. Die Zellen wurden für 20 min im Dunkeln mit dem Färbepuffer inkubiert, bevor sie in Probenmessgefäße für das FACS Scan überführt werden. Die DNA Messung erfolgte in einem Becton Dickinson FACS Analyzer, mit einen Argonlaser (500 mW, Emission 488 nm), und dem DNA Cell Quest Programm (BD). Die logarithmische PI Fluoreszenz wurde mit einem band-pass Filter (BP 585/42) bestimmt. Die Quantifizierung der Zellpopulationen in den einzelnen Zellzyklusphasen erfolgte mit dem ModFit LT Programm von Becton Dickinson.

Entsprechend, wurden erfindungsgemäße Verbindungen auf weiteren Tumorzellen getestet. Beispielsweise sind diese Verbindungen auf Karzinomen verschiedenster Gewebe (z. Bspl. Brust (MCF7); Colon (HCT116), Kopf-Hals (FaDu), Lunge (NCl-H460), Pankreas (BxPC-3), Prostata (DU145)), Sarkome (z. Bspl. SK-UT-1 B), Leukämien und Lymphome (z. Bspl. HL-60; Jurkat, THP-1) und anderen Tumoren (z. Bspl. Melanome (BRO), Gliome (U-87MG)) aktiv und könnten in solchen Indikationen eingesetzt werden. Dies belegt die breite Anwendbarkeit der erfindungsgemäßen Verbindungen zur Behandlung verschiedenster Tumortypen.

Die Verbindungen der allgemeinen Formel (I) können allein oder in Kombination mit anderen erfindungsgemäßen Wirkstoffen, gegebenenfalls auch in Kombination mit weiteren pharmakologisch aktiven Wirkstoffen, zur Anwendung gelangen.

Geeignete Anwendungsformen sind beispielsweise Tabletten, Kapseln, Zäpfchen, Lösungen, - insbesondere Lösungen zur Injektion (s.c., i.v., i.m.) und Infusion - Säfte, Emulsionen oder dispersible Pulver. Hierbei soll der Anteil der pharmazeutisch wirksamen Verbindung(en) jeweils im Bereich von 0,1 - 90 Gew.- %, bevorzugt 0,5 - 50 Gew.-% der Gesamtzusammensetzung liegen, d.h. in Mengen die ausreichend sind, um den unten angegebenen Dosierungsbereich zu erreichen. Die genannten Dosen können, falls erforderlich, mehrmals täglich gegeben werden.

Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektions- und Infusionslösungen werden in üblicher Weise, z.B. unter Zusatz von Isotonantien, Konservierungsmitteln, wie p-Hydroxybenzoaten, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösemittel als Lösevermittler bzw. Hilflösemittel eingesetzt werden können, hergestellt und in Injektionsflaschen oder Ampullen oder Infusionsflaschen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuß- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Die Applikation erfolgt in üblicher Weise, vorzugsweise oral oder transdermal, insbesondere bevorzugt oral. Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden. Die Dosierung für die intravenöse Anwendung liegt bei 1 - 1000 mg pro Stunde, vorzugsweise zwischen 5 - 500 mg pro Stunde.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über der Tag zu verteilen.

Die nachfolgenden Formulierungsbeispiele illustrieren die vorliegende Erfindung ohne sie jedoch in ihrem Umfang zu beschränken:

### Pharmazeutische Formulierungsbeispiele

| A) | Tabletten | pro Tablette |
|---|---|---|
| | Wirkstoff | 100 mg |
| | Milchzucker | 140 mg |
| | Maisstärke | 240 mg |
| | Polyvinylpyrrolidon | 15 mg |
| | Magnesiumstearat | 5 mg |
| | | 500 mg |

Der feingemahlene Wirkstoff, Milchzucker und ein Teil der Maisstärke werden miteinander vermischt. Die Mischung wird gesiebt, danach mit einer Lösung von Polyvinylpyrrolidon in Wasser befeuchtet, geknetet, feuchtgranuliert und getrocknet. Das Granulat, der Rest der Maisstärke und das Magnesiumstearat werden gesiebt und miteinander vermischt. Das Gemisch wird zu Tabletten geeigneter Form und Größe verpreßt.

| B) | Tabletten | pro Tablette |
|---|---|---|
| | Wirkstoff | 80 mg |
| | Milchzucker | 55 mg |
| | Maisstärke | 190 mg |
| | Mikrokristalline Cellulose | 35 mg |
| | Polyvinylpyrrolidon | 15 mg |
| | Natrium-carboxymethylstärke | 23 mg |
| | Magnesiumstearat | 2 mg |
| | | 400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natriumcarboxymethylstärke und das Magnesiumstearat, vermischt und verpreßt das Gemisch zu Tabletten geeigneter Größe.

| C) | Ampullenlösung | |
|---|---|---|
| | Wirkstoff | 50 mg |
| | Natriumchlorid | 50 mg |
| | Aqua pro inj. | 5 ml |

Der Wirkstoff wird bei Eigen-pH oder gegebenenfalls bei pH 5,5 - 6,5 in Wasser gelöst und mit Natriumchlorid als Isotonans versetzt. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

## Patentansprüche

1. Verbindungen der allgemeinen Formel (I), 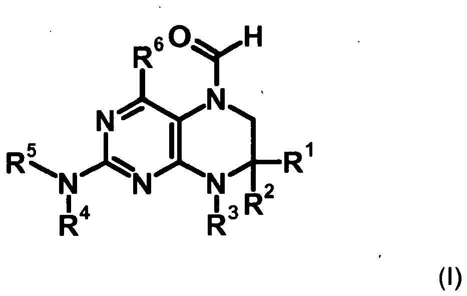 worin
R¹, R² gleich oder verschieden ein Rest ausgewählt aus der Gruppe bestehend aus gegebenenfalls substituiertem C₁-C₁₀-Alkyl, C₂-C₁₀-Alkenyl, C₂-C₁₀-Alkinyl, Aryl, Heteroaryl, C₃-C₈-Cycloalkyl, C₃-C₈-Heterocycloalkyl, -X-Aryl, -X-Heteroaryl, -X-Cycloalkyl, -X-Heterocycloalkyl, -NR⁷-Aryl, -NR⁷-Heteroaryl, -NR⁷-Cycloalkyl und -NR⁷-Heterocycloalkyl,
wobei die oben genannten Alkyl, Alkenyl und Alkinylreste gegebenenfalls mit Methyl, Chlor oder Fluor substituiert sein können, und
wobei die oben genannten Arylreste gegebenenfalls mit OH, NO₂, CN, OMe, - OCHF₂, -OCF₃, -NH₂, Halogen, C₁-C₁₀-Alkyl, -O-C₁-C₃-Alkyl, -COOH, -COO-C₁-C₄-Alkyl, oder -CONH₂ substituiert sein können, und
wobei die oben genannten Heteroarylreste einen oder mehrere der nachfolgend genannten Substituenten tragen können:
a) F, Cl, Br, OH, OMe, Methyl, Ethyl, CN, CONH₂, NH₂,
b) gegebenenfalls mit OH, NO₂, CN, OMe, -OCHF₂, -OCF₃, -NH₂, Halogen, C₁-C₁₀-Alkyl, -O-C₁-C₃-Alkyl, -COOH, -COO-C₁-C₄-Alkyl, oder -CONH₂ substituiertes Phenyl,
c) gegebenenfalls mit F, Cl, Br, OH, OMe, Methyl, Ethyl, CN, CONH₂ oder NH₂ substituiertes Heteroaryl
d) oder mit gegebenenfalls mit OH, NO₂, CN, OMe, -OCHF₂, -OCF₃, -NH₂, Halogen, C₁-C₁₀-Alkyl, -O-C₁-C₃-Alkyl, -COOH, -COO-C₁-C₄-Alkyl, oder -CONH₂ substituiertem Phenyl substituiertes Heteroaryl, und
wobei die oben genannten Cycloalkylreste gegebenenfalls mit OH, NO₂, CN, OMe, - OCHF₂, -OCF₃, -NH₂ oder Halogen, C₁-C₁₀-Alkyl, -O-C₁-C₃-Alkyl, -COOH, -COO-C₁-C₄-Alkyl oder -CONH₂ substituiert sein können, und
wobei die oben genannten Heterocycloalkylreste gegebenenfalls mit C₁-C₄-Alkyl, substituiert sein können,
oder
ein Rest ausgewählt aus der Gruppe bestehend aus Wasserstoff, Halogen, COXR⁷, CON(R⁷)₂, COR⁷ und XR⁷,
oder
R¹ und R² gemeinsam eine 2- bis 5-gliedrige Alkylbrücke, die 1 bis 2 Heteroatome enthalten kann,
R³ Wasserstoff oder ein Rest ausgewählt aus der Gruppe bestehend aus gegebenenfalls substituiertem C₁-C₁₂-Alkyl, C₂-C₁₂-Alkenyl , C₂-C₁₂-Alkinyl, Aryl, Heteroaryl, -C₃-C₁₂-Cycloalkyl, C₃-C₁₂-Cycloalkenyl, C₇-C₁₂-Polycycloalkyl, C₇-C₁₂-Polycycloalkenyl und C₅-C₁₂-Spirocycloalkyl
wobei die oben genannten Alkyl, Alkenyl und Alkinylreste gegebenenfalls mit Methyl, Chlor oder Fluor substituiert sein können, und
wobei die oben genannten Arylreste gegebenenfalls mit OH, NO₂, CN, OMe, - OCHF₂, -OCF₃, -NH₂, Halogen, C₁-C₁₀-Alkyl, -O-C₁-C₃-Alkyl, -COOH, -COO-C₁-C₄-Alkyl, oder -CONH₂ substituiert sein können, und
wobei die oben genannten Heteroarylreste einen oder mehrere der nachfolgend genannten Substituenten tragen können:
a) F, Cl, Br, OH, OMe, Methyl, Ethyl, CN, CONH₂, NH₂,
b) gegebenenfalls mit OH, NO₂, CN, OMe, -OCHF₂, -OCF₃, -NH₂, Halogen, C₁-C₁₀-Alkyl, -O-C₁-C₃-Alkyl, -COOH, -COO-C₁-C₄-Alkyl, oder -CONH₂ substituiertes Phenyl,
c) gegebenenfalls mit F, Cl, Br, OH, OMe, Methyl, Ethyl, CN, CONH₂ oder NH₂ substituiertes Heteroaryl
d) oder mit gegebenenfalls mit OH, NO₂, CN, OMe, -OCHF₂, -OCF₃, -NH₂, Halogen, C₁-C₁₀-Alkyl, -O-C₁-C₃-Alkyl, -COOH, -COO-C₁-C₄-Alkyl, oder -CONH₂ substituiertem Phenyl substituiertes Heteroaryl, und
wobei die oben genannten Cycloalkylreste gegebenenfalls mit OH, NO₂, CN, OMe, - OCHF₂, -OCF₃, -NH₂ oder Halogen, C₁-C₁₀-Alkyl, -O-C₁-C₃-Alkyl, -COOH, -COO-C₁-C₄-Alkyl oder -CONH₂ substituiert sein können,
oder
R¹ und R³ oder R² und R³ gemeinsam eine gesättigte oder ungesättigte C₃-C₄-Alkylbrücke, die 1 bis 2 Heteroatome enthalten kann,
R⁴ gegebenenfalls substituiertes Aryl, Benzyl oder Heteroaryl,
R⁵ Wasserstoff, -CO-NH-C₁-C₄-Alkyl, -CO-C₁-C₄-Alkyl oder -CO-X-C₁-C₄-Alkyl,
R⁶ ein Rest ausgewählt aus der Gruppe bestehend aus Wasserstoff, NH₂, XH, Halogen und einer gegebenenfalls durch ein oder mehrere Halogenatome substituierten C₁-C₃-Alkyl-Gruppe,
und
R⁷ jeweils unabhängig voneinander, Wasserstoff oder ein Rest ausgewählt aus der Gruppe bestehend aus gegebenenfalls substituiertem C₁-C₄-Alkyl, C₂-C₄-Alkenyl, C₂-C₄-Alkinyl, Benzyl und Phenyl,
wobei die oben genannten Alkyl, Alkenyl und Alkinylreste gegebenenfalls mit Methyl, Chlor oder Fluor substituiert sein können, und
wobei die oben genannten Arylreste gegebenenfalls mit OH, NO₂, CN, OMe, - OCHF₂, -OCF₃, -NH₂, Halogen, C₁-C₁₀-Alkyl, -O-C₁-C₃-Alkyl, -COOH, -COO-C₁-C₄-Alkyl, oder -CONH₂ substituiert sein können,
X O oder S
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze, Solvate oder Hydrate,
bedeuten.

2. Verbindungen nach Anspruch 1,
worin
R¹ bis R⁴ und R⁷ die angegebene Bedeutung aufweisen und
R⁵ und R⁶ Wasserstoff
bedeuten.

3. Verbindungen nach Anspruch 1 oder 2,
worin
R³ bis R⁷ die angegebene Bedeutung aufweisen und
R¹, R² gleich oder verschieden, Wasserstoff oder ein Rest, ausgewählt aus der Gruppe bestehend aus gegebenenfalls substituiertem C₁-C₆-Alkyl, C₂-C₆-Alkenyl, und C₂-C₆-Alkinyl,
wobei die oben genannten Alkyl, Alkenyl und Alkinylreste gegebenenfalls mit Methyl, Chlor oder Fluor substituiert sein können,
oder
R¹ und R² gemeinsam eine 2- bis 5-gliedrige Alkylbrücke
bedeuten.

4. Verbindungen nach einem der Ansprüche 1 bis 3,
worin
R¹, R² und R⁴ bis R⁷ die angegebene Bedeutung aufweisen,
und
R³ gleich Wasserstoff oder ein Rest, ausgewählt aus der Gruppe bestehend aus gegebenenfalls substituiertem C₁-C₁₂-Alkyl, C₂-C₁₂-Alkenyl, C₂-C₁₂-Alkinyl und C₆-C₁₄-Aryl,
wobei die oben genannten Alkyl, Alkenyl und Alkinylreste gegebenenfalls mit Methyl, Chlor oder Fluor substituiert sein können, und
wobei die oben genannten Arylreste gegebenenfalls mit OH, NO₂, CN, OMe, - OCHF₂, -OCF₃, -NH₂, Halogen, C₁-C₁₀-Alkyl, -O-C₁-C₃-Alkyl, -COOH, -COO-C₁-C₄-Alkyl, oder -CONH₂ substituiert sein können,
oder
ein Rest ausgewählt aus der Gruppe bestehend aus gegebenenfalls substituiertem C₃-C₁₂-Cycloalkyl, C₃-C₁₂-Cycloalkenyl, C₇-C₁₂-Polycydoalkyl, C₇-C₁₂-Polycycloalkenyl und C₅-C₁₂-Spirocycloalkyl,
wobei die oben genannten Cycloalkylreste gegebenenfalls mit OH, NO₂, CN, OMe, - OCHF₂, -OCF₃, -NH₂ oder Halogen, C₁-C₁₀-Alkyl, -O-C₁-C₃-Alkyl, -COOH, -COO-C₁-C₄-Alkyl oder -CONH₂ substituiert sein können,
bedeutet.

5. Verbindungen nach einem der Ansprüche 1 bis 4,
worin
R¹ bis R³ und R⁵ bis R⁷ die angegebene Bedeutung aufweisen,
und
R⁴ ein Rest der allgemeinen Formel 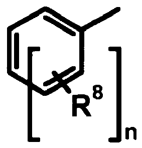 R⁸ gleich oder verschieden, Wasserstoff oder ein Rest ausgewählt aus der Gruppe bestehend aus gegebenenfalls substituiertem C₁-C₆-Alkyl, C₂-C₆-Alkenyl, C₂-C₆-Alkinyl, -O-C₁-C₆-Alkyl, -O-C₂-C₆-Alkenyl, -O-C₂-C₆-Alkinyl, Heterocycloalkyl, C₃-C₆-Cycloalkyl, Aryl, Heteroaryl, -O-Aryl, -O-Heteroaryl, -O-Cycloalkyl, und -O-Heterocycloal kyl
wobei die oben genannten Alkyl, Alkenyl und Alkinylreste gegebenenfalls mit Methyl, Chlor oder Fluor substituiert sein können, und
wobei die oben genannten Arylreste gegebenenfalls mit OH, NO₂, CN, OMe, - OCHF₂, -OCF₃, -NH₂, Halogen, C₁-C₁₀-Alkyl, -O-C₁-C₃-Alkyl, -COOH, -COO-C₁-C₄-Alkyl, oder -CONH₂ substituiert sein können, und
wobei die oben genannten Heteroarylreste einen oder mehrere der nachfolgend genannten Substituenten tragen können:
a) F, Cl, Br, OH, OMe, Methyl, Ethyl, CN, CONH₂, NH₂,
b) gegebenenfalls mit OH, NO₂, CN, OMe, -OCHF₂, -OCF₃, -NH₂, Halogen, C₁-C₁₀-Alkyl, -O-C₁-C₃-Alkyl, -COOH, -COO-C₁-C₄-Alkyl, oder -CONH₂ substituiertes Phenyl,
c) gegebenenfalls mit F, Cl, Br, OH, OMe, Methyl, Ethyl, CN, CONH₂ oder NH₂ substituiertes Heteroaryl
d) oder mit gegebenenfalls mit OH, NO₂, CN, OMe, -OCHF₂, -OCF₃, -NH₂, Halogen, C₁-C₁₀-Alkyl, -O-C₁-C₃-Alkyl, -COOH, -COO-C₁-C₄-Alkyl, oder -CONH₂ substituiertem Phenyl substituiertes Heteroaryl, und
wobei die oben genannten Cycloalkylreste gegebenenfalls mit OH, NO₂, CN, OMe, - OCHF₂, -OCF₃, -NH₂ oder Halogen, C₁-C₁₀-Alkyl, -O-C₁-C₃-Alkyl, -COOH, -COO-C₁-C₄-Alkyl oder -CONH₂ substituiert sein können,
oder
ein Rest ausgewählt aus der Gruppe bestehend aus Wasserstoff, -CONH₂, -COOR⁷,-OCON(R⁷)₂, -N(R⁷)₂, -NHCOR⁷, -NHCON(R⁷)₂, -NO₂, CF₃, Halogen, -O-C₁-C₆-Alkyl-Q¹, -CONR⁷-C₁-C₁₀-Alkyl-Q¹, -CONR⁷-C₁-C₁₀-Alkenyl-Q¹, -CONR⁷-Q², Halogen, OH, -SO₂R⁷, -SO₂N(R⁷)₂, -COR⁷, -COOR⁷, -N(R⁷)₂, -NHCOR⁷, -CONR⁷OC₁-C₁₀ -Alkyl-Q¹ und CONR⁷O-Q²,
oder
benachbarte Reste R⁸ gemeinsam eine Brücke der allgemeinen Formel a), b), c) oder d), 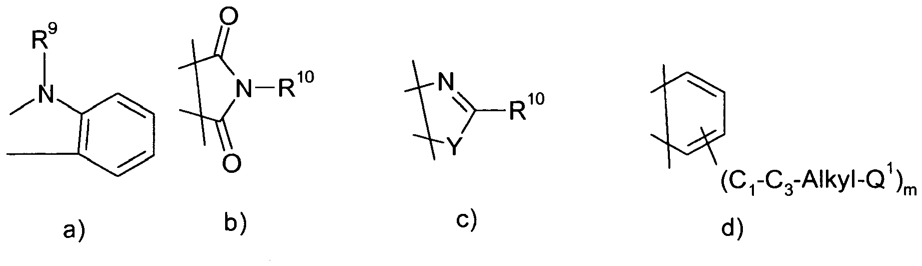 Y O, S oder NR¹¹,
m 0,1 oder 2
R⁹ C₁-C₆-Alkyl
R¹⁰ Wasserstoff oder ein Rest ausgewählt aus der Gruppe bestehend aus gegebenenfalls substituiertem Phenyl, Pyridyl, Pyrazinyl, Pyrimidinyl, Pyridazinyl, Piperidinyl, Piperazinyl, -C₁-C₃-Alkyl-Phenyl, -C₁-C₃-Alkyl-Pyridyl, -C₁-C₃-Alkyl-Pyrazinyl, -C₁-C₃-Alkyl-Pyrimidinyl und -C₁-C₃-Alkyl-Pyridazinyl,
wobei die oben genannten Alkyl, Alkenyl und Alkinylreste gegebenenfalls mit Methyl, Chlor oder Fluor substituiert sein können, und
wobei die oben genannten Arylreste gegebenenfalls mit OH, NO₂, CN, OMe, - OCHF₂, -OCF₃, -NH₂, Halogen, C₁-C₁₀-Alkyl, -O-C₁-C₃-Alkyl -COOH, -COO-C₁-C₄-Alkyl, oder -CONH₂ substituiert sein können, und
wobei die oben genannten Heteroarylreste einen oder mehrere der nachfolgend genannten Substituenten tragen können:
a) F, Cl, Br, OH, OMe, Methyl, Ethyl, CN, CONH₂, NH₂,
b) gegebenenfalls mit OH, NO₂, CN, OMe, -OCHF₂, -OCF₃, -NH₂, Halogen, C₁-C₁₀-Alkyl, -O-C₁-C₃-Alkyl, -COOH, -COO-C₁-C₄-Alkyl, oder -CONH₂ substituiertes Phenyl,
c) gegebenenfalls mit F, Cl, Br, OH, OMe, Methyl, Ethyl, CN, CONH₂ oder NH₂ substituiertes Heteroaryl
d) oder mit gegebenenfalls mit OH, NO₂, CN, OMe, -OCHF₂, -OCF₃, -NH₂, Halogen, C₁-C₁₀-Alkyl, -O-C₁-C₃-Alkyl, -COOH, -COO-C₁-C₄-Alkyl, oder -CONH₂ substituiertem Phenyl substituiertes Heteroaryl, und
wobei die oben genannten Cycloalkylreste gegebenenfalls mit OH, NO₂, CN, OMe, - OCHF₂, -OCF₃, -NH₂ oder Halogen, C₁-C₁₀-Alkyl, -O-C₁-C₃-Alkyl, -COOH, -COO-C₁-C₄-Alkyl oder -CONH₂ substituiert sein können,
R¹¹ Wasserstoff oder C¹-C⁴-Alkyl
Q¹ Wasserstoff, -NHCOR⁷, oder ein Rest ausgewählt aus der Gruppe bestehend aus einer gegebenenfalls substituierten -NH-Aryl, -NH-Heteroaryl, Aryl-, Heteroaryl-, C₃-C₈-Cycloalkyl- und Heterocycloalkyl-Gruppe,
wobei die oben genannten Arylreste gegebenenfalls mit OH, NO₂, CN, OMe, - OCHF₂, -OCF₃, -NH₂, Halogen, C₁-C₁₀-Alkyl, -O-C₁-C₃-Alkyl, -COOH, -COO-C₁-C₄-Alkyl, oder -CONH₂ substituiert sein können, und
wobei die oben genannten Heteroarylreste einen oder mehrere der nachfolgend genannten Substituenten tragen können:
a) F, Cl, Br, OH, OMe, Methyl, Ethyl, CN, CONH₂, NH₂,
b) gegebenenfalls mit OH, NO₂, CN, OMe, -OCHF₂, -OCF₃, -NH₂, Halogen, C₁-C₁₀-Alkyl, -O-C₁-C₃-Alkyl, -COOH, -COO-C₁-C₄-Alkyl, oder -CONH₂ substituiertes Phenyl,
c) gegebenenfalls mit F, Cl, Br, OH, OMe, Methyl, Ethyl, CN, CONH₂ oder NH₂ substituiertes Heteroaryl
d) oder mit gegebenenfalls mit OH, NO₂, CN, OMe, -OCHF₂, -OCF₃, -NH₂, Halogen, C₁-C₁₀-Alkyl, -O-C₁-C₃-Alkyl, -COOH, -COO-C₁-C₄-Alkyl, oder -CONH₂ substituiertem Phenyl substituiertes Heteroaryl, und
wobei die oben genannten Cycloalkylreste gegebenenfalls mit OH, NO₂, CN, OMe, - OCHF₂, -OCF₃, -NH₂ oder Halogen, C₁-C₁₀-Alkyl, -O-C₁-C₃-Alkyl, -COOH, -COO-C₁-C₄-Alkyl oder -CONH₂ substituiert sein können,
Q² Wasserstoff oder ein Rest ausgewählt aus der Gruppe bestehend aus einer gegebenenfalls substituierten Aryl-, Heteroaryl-, C₃-C₈-Heterocycloalkyl, C₃-C₈-Cycloalkyl- und C₁-C₄-Alkyl-C₃-C₈-cycloalkyl-Gruppe,
wobei die oben genannten Arylreste gegebenenfalls mit OH, NO₂, CN, OMe, - OCHF₂, -OCF₃, -NH₂, Halogen, C₁-C₁₀-Alkyl, -O-C₁-C₃-Alkyl, -COOH, -COO-C₁-C₄-Alkyl, oder -CONH₂ substituiert sein können, und
wobei die oben genannten Heteroarylreste einen oder mehrere der nachfolgend genannten Substituenten tragen können:
a) F, Cl, Br, OH, OMe, Methyl, Ethyl, CN, CONH₂, NH₂,
b) gegebenenfalls mit OH, NO₂, CN, OMe, -OCHF₂, -OCF₃, -NH₂, Halogen, C₁-C₁₀-Alkyl, -O-C₁-C₃-Alkyl, -COOH, -COO-C₁-C₄-Alkyl, oder -CONH₂ substituiertes Phenyl,
c) gegebenenfalls mit F, Cl, Br, OH, OMe, Methyl, Ethyl, CN, CONH₂ oder NH₂ substituiertes Heteroaryl
d) oder mit gegebenenfalls mit OH, NO₂, CN, OMe, -OCHF₂, -OCF₃, -NH₂, Halogen, C₁-C₁₀-Alkyl, -O-C₁-C₃-Alkyl, -COOH, -COO-C₁-C₄-Alkyl, oder -CONH₂ substituiertem Phenyl substituiertes Heteroaryl, und
wobei die oben genannten Cycloalkylreste gegebenenfalls mit OH, NO₂, CN, OMe, - OCHF₂, -OCF₃, -NH₂ oder Halogen, C₁-C₁₀-Alkyl, -O-C₁-C₃-Alkyl, -COOH, -COO-C₁-C₄-Alkyl oder -CONH₂ substituiert sein können,
und
n 0, 1, 2, 3, 4 oder 5,
bedeuten.

6. Verbindungen nach einem der Ansprüche 1 bis 5,
worin
Q¹, Q², n, R⁴ bis R⁸ die angegebene Bedeutung aufweisen,
R¹, R² gleich oder verschieden, Wasserstoff oder ein Rest ausgewählt aus der Gruppe bestehend Methyl, Ethyl, Propyl, Allyl und Propargyl
oder
R¹ und R² gemeinsam Cyclopropyl,
R³ gleich Wasserstoff, oder gegebenenfalls substituiertes C₁-C₆-Alkyl oder gegebenenfalls substituiertes C₃-C₁₂-Cycloalkyl,
wobei die oben genannten Alkylreste gegebenenfalls mit Methyl, Chlor oder Fluor substituiert sein können, und
wobei die oben genannten Cycloalkylreste gegebenenfalls mit OH, NO₂, CN, OMe, - OCHF₂, -OCF₃, -NH₂ oder Halogen, C₁-C₁₀-Alkyl, -O-C₁-C₃-Alkyl, -COOH, -COO-C₁-C₄-Alkyl oder -CONH₂ substituiert sein können,
bedeuten.

7. Verbindungen nach Anspruch 5 oder 6,
worin
Q¹, Q², n, R¹ bis R⁴, R⁶ bis R⁷ die angegebene Bedeutung aufweisen,
und
R⁸ gleich oder verschieden, Wasserstoff oder einen Rest ausgewählt aus der Gruppe bestehend aus Halogen, (C₁-C₂-Alkyl)₂N-, CF₃, NH₂SO₂-, -CONH-C₆-C₁₄-Aryl, - CONH-C₁-C₄-Alkyl-C₆-C₁₄-aryl, -CONH-C₁-C₄-Alkyl-C₆-C₁₄-heteroaryl, -CONH- C₃-C₈-cycloalkyl-Heterocycloalkyl und -O-C₁-C₄-Alkyl,
bedeuten.

8. Verbindung der Formel (I) nach einem der Ansprüche 1 bis 7 zur Verwendung als Arzneimittel.

9. Verbindung der Formel (I) nach einem der Ansprüche 1 bis 7 zur Verwendung als Arzneimittel mit antiproliferativer Wirkung.

10. Verwendung einer Verbindung der Formel (I) gemäß einem der Ansprüche 1 bis 7 zur Herstellung eines Arzneimittels zur Behandlung und/oder Prävention von Erkrankungen ausgewählt aus der Gruppe bestehend aus Krebs, bakteriellen und viralen Infektionen, Entzündungs- und Autoimmunerkrankungen, Chemotherapeutika induzierter Alopezie und Mukositis, kardiovaskulärer Erkrankungen, nephrologischen Erkrankungen, sowie chronisch und akut neurodegenerativen Erkrankungen.

11. Verwendung einer Verbindung der Formel (I) gemäß einem der Ansprüche 1 bis 7 zur Herstellung eines Arzneimittels zur Inhibitierung der Polo-like Kinasen.

12. Verwendung gemäß Anspruch 11, **dadurch gekennzeichnet, dass** die Polo-like Kinase PLK1 ist.

13. Verwendung einer Verbindung der Formel (I) gemäß einem der Ansprüche 1 bis 7 zur Herstellung eines Arzneimittels zur Behandlung und/oder Prävention von auf Überexpression der Polo-like Kinasen, beruhenden Tumorerkrankungen.

14. Pharmazeutische Zubereitungen, enthaltend als Wirkstoff eine oder mehrere Verbindungen der allgemeinen Formel (I) gemäß einem der Ansprüche 1 bis 7 gegebenenfalls in Kombination mit üblichen Hilfs- und/oder Trägerstoffen.

## Claims

1. Compounds of general formula (I), 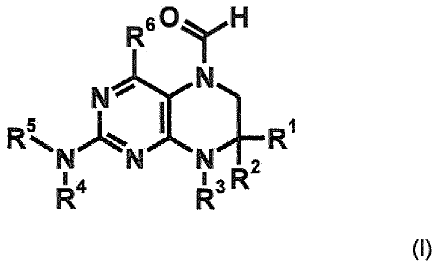 wherein
R¹, R² which may be identical or different denote a group selected from among optionally substituted C₁-C₁₀-alkyl, C₂-C₁₀-alkenyl, C₂-C₁₀-alkynyl, aryl, heteroaryl, C₃-C₈-cycloalkyl, C₃-C₈-heterocycloalkyl, -X-aryl, -X-heteroaryl, -X-cycloalkyl, -X-heterocycloalkyl, -NR⁷-aryl, -NR⁷-heteroaryl, -NR⁷-cycloalkyl and -NR⁷-heterocycloalkyl,
wherein the above-mentioned alkyl, alkenyl and alkynyl groups may optionally be substituted by methyl, chlorine or fluorine, and
wherein the above-mentioned aryl groups may optionally be substituted by OH, NO₂, CN, OMe, -OCHF₂, -OCF₃, -NH₂, halogen, C₁-C₁₀-alkyl, -O-C₁-C₃-alkyl, -COOH, -COO-C₁-C₄-alkyl, or -CONH₂, and
wherein the above-mentioned heteroaryl, groups may carry one or more of the substituents named below:
a) F, Cl, Br, OH, OMe, methyl, ethyl, CN, CONH₂, NH₂,
b) phenyl optionally substituted by OH, NO₂, CN, OMe, -OCHF₂, -OCF₃, -NH₂, halogen, C₁-C₁₀-alkyl, -O-C₁-C₃-alkyl, -COOH, -COO-C₁-C₄-alkyl, or -CONH₂,
c) heteroaryl optionally substituted by F, Cl, Br, NH, OMe, methyl, ethyl, CN, CONH₂ or NH₂,
d) or heteroaryl substituted by phenyl optionally substituted by OH, NO₂, CN, OMe, -OCHF₂, -OCF₃, -NH₂, halogen, C₁-C₁₀-alkyl, -O-C₁-C₃-alkyl, -COOH, -COO-C₁-C₄-alkyl, or -CONH₂, and
wherein the above-mentioned cycloalkyl groups may optionally be substituted by OH, NO₂, CN, OMe, -OCHF₂, -OCF₃, -NH₂ or halogen, C₁-C₁₀-alkyl, -O-C₁-C₃-alkyl, -COOH, -COO-C₁-C₄-alkyl, or -CONH₂, and
wherein the above-mentioned heterocycloalkyl groups may optionally be substituted by C₁-C₄-alkyl,
or
a group selected from among hydrogen, halogen, COXR⁷, CON(R⁷)₂, COR⁷ and XR⁷,
or
R¹ and R² together denote a 2- to 5-membered alkyl bridge which may contain 1 to 2 heteroatoms,
R³ denotes hydrogen or a group selected from among optionally substituted C₁-C₁₂-alkyl, C₂-C₁₂-alkenyl, C₂-C₁₂₋alkynyl, aryl, heteroaryl, -C₃-C₁₂-cycloalkyl, C₃-C₁₂-cycloalkenyl, C₇-C₁₂-polycycloalkyl, C₇-C₁₂-polycycloalkenyl and C₅-C₁₂-spirocycloalkyl
wherein the above-mentioned alkyl, alkenyl and alkynyl groups may optionally be substituted by methyl, chlorine or fluorine, and
wherein the above-mentioned aryl groups may optionally be substituted by OH, NO₂, CN, OMe, -OCHF₂, -OCHF₃, -NH₂, halogen, C₁-C₁₀-alkyl, -O-C₁-C₃-alkyl, -COOH, -COO-C₁-C₄-alkyl, or -CONH₂, and
wherein the above-mentioned heteroaryl groups may carry one or more of the substituents named below:
a) F, Cl, Br, NH, OMe, methyl, ethyl, CN, CONH₂, NH₂,
b) phenyl optionally substituted by OH, NO₂, CN, OMe, -OCHF₂, -OCF₃, -NH₂, halogen, C₁-C₁₀-alkyl, -O-C₁-C₃-alkyl, -COOH, -COO-C₁-C₄-alkyl, or -CONH₂,
c) heteroaryl optionally substituted by F, Cl, Br, OH, OMe, methyl, ethyl, CN, CONH₂ or NH₂,
d) or heteroaryl substituted by phenyl optionally substituted by OH, NO₂, CN, OMe, -OCHF₂, -OCF₃, -NH₂, halogen, C₁-C₁₀-alkyl, -O-C₁-C₃-alkyl, -COOH, -COO-C₁-C₄-alkyl, or -CONH₂, and
wherein the above-mentioned cycloalkyl groups may optionally be substituted by OH, NO₂, CN, OMe, -OCHF₂, -OCF₃, -NH₂ or halogen, C₁-C₁₀-alkyl, -O-C₁-C₃-alkyl, -COOH, -COO-C₁-C₄-alkyl, or -CONH₂,
or
R¹ and R³ or R² and R³ together denote a saturated or unsaturated C₃-C₄-alkyl bridge which may contain 1 to 2 heteroatoms,
R⁴ denotes optionally substituted aryl, benzyl or heteroaryl,
R⁵ denotes hydrogen, -CO-NH-C₁-C₄-alkyl, -CO-C₁-C₄-alkyl or -CO-X-C₁-C₄-alkyl,
R⁶ denotes a group selected from among hydrogen, NH₂, XH, halogen and a C₁-C₃-alkyl group optionally substituted by one or more halogen atoms,
and
R⁷ each independently of one another denote hydrogen or a group selected from among optionally substituted C₁-C₄-alkyl, C₂-C₄-alkenyl, C₂-C₄-alkynyl, benzyl and phenyl,
wherein the above-mentioned alkyl, alkenyl and alkynyl groups may optionally be substituted by methyl, chlorine or fluorine, and
wherein the above-mentioned aryl groups may optionally be substituted by OH, NO₂, CN, OMe, -OCHF₂, -OCF₃, -NH₂, halogen, C₁-C₁₀-alkyl, -O-C₁-C₃-alkyl, -COOH, -COO-C₁-C₄-alkyl, or -CONH₂,
X denotes O or S,
optionally in the form of the tautomers, racemates, enantiomers, diastereomers and mixtures thereof, and optionally the pharmacologically acceptable acid addition salts, solvates or hydrates thereof.

2. Compounds according to claim 1,
wherein
R¹ to R⁴ and R⁷ are as hereinbefore defined and
R⁵ and R⁶ represent hydrogen.

3. Compounds according to claim 1 or 2,
wherein
R³ to R⁷ are as hereinbefore defined and
R¹, R² which may be identical or different denote hydrogen or a group selected from among optionally substituted C₁-C₆-alkyl, C₂-C₆-alkenyl, and C₂-C₆-alkynyl,
wherein the above-mentioned alkyl, alkenyl and alkynyl groups may optionally be substituted by methyl, chlorine or fluorine,
or
R¹ and R² together denote a 2- to 5-membered alkyl bridge.

4. Compounds according to one of claims 1 to 3,
wherein
R¹, R² and R⁴ to R⁷ are as hereinbefore defined,
and
R³ is hydrogen or a group selected from among optionally substituted C₁-C₁₂-alkyl, C₂-C₁₂-alkenyl, C₂-C₁₂-alkynyl and C₆-C₁₄-aryl,
wherein the above-mentioned alkyl, alkenyl and alkynyl groups may optionally be substituted by methyl, chlorine or fluorine, and
wherein the above-mentioned aryl groups may optionally be substituted by OH, NO₂, CN, OMe, -OCHF₂, -OCF₃, -NH₂, halogen, C₁-C₁₀-alkyl, -O-C₁-C₃-alkyl, -COOH, -COO-C₁-C₄-alkyl, or -CONH₂,
or
a group selected from among optionally substituted C₃-C₁₂-cycloalkyl, C₃-C₁₂-cycloalkenyl, C₇-C₁₂-polycycloalkyl, C₇-C₁₂-polycycloalkenyl and C₅-C₁₂-spirocycloalkyl,
wherein the above-mentioned cycloalkyl groups may optionally be substituted by OH, NO₂, CN, OMe, -OCHF₂, -OCF₃, -NH₂ or halogen, C₁-C₁₀-alkyl, -O-C₁-C₃-alkyl, -COOH, -COO-C₁-C₄-alkyl or -CONH₂.

5. Compounds according to one of claims 1 to 4, wherein
R¹ to R³ and R⁵ to R⁷ are as hereinbefore defined, and
R⁴ denotes a group of general formula 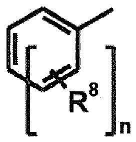 R⁸ which may be identical or different denotes hydrogen or a group selected from among optionally substituted C₁-C₆-alkyl, C₂-C₆-alkenyl, C₂-C₆-alkynyl, -O-C₁-C₆-alkyl, -O-C₂-C₆-alkenyl -O-C₂-C₆-alkynyl, heterocycloalkyl, C₃-C₆-cycloalkyl, aryl, heteroaryl, -O-aryl, -O-heteroaryl, -O-cycloalkyl, and -O-heterocycloalkyl,
wherein the above-mentioned alkyl, alkenyl and alkenyl groups may optionally be substituted by methyl, chlorine or fluorine, and
wherein the above-mentioned aryl groups may optionally be substituted by OH, NO₂, CN, OMe, -OCHF₂, -OCF₃, -NH₂, halogen, C₁-C₁₀-alkyl, -O-C₁-C₃-alkyl, -COOH, -COO-C₁-C₄-alkyl, or -CONH₂, and
wherein the above-mentioned heteroaryl groups may carry one or more of the substituents named below:
a) F, Cl, Br, OH, OMe, methyl, ethyl, CN, CONH₂, NH₂,
b) phenol optionally substituted by OH, NO₂, CN, OMe, -OCHF₂, -OCF₃, -NH₂, halogen, C₁-C₁₀-alkyl, -O-C₁-C₃-alkyl, -COOH, -COO-C₁-C₄-alkyl, or -CONH₂,
c) heteroaryl optionally substituted by F, Cl, Br, OH, OMe, methyl, ethyl, CN, CONH₂ or NH₂,
d) or heteroaryl substituted by phenol optionally substituted by OH, NO₂, CN, OMe, -OCHF₂, -OCF₃, -NH₂, halogen, C₁-C₁₀-alkyl, -O-C₁-C₃-alkyl, -COOH, -COO-C₁-C₄-alkyl, or -CONH₂, and
wherein the above-mentioned cycloalkyl groups may optionally be substituted by OH, NO₂, CN, OMe, -OCHF₂, -OCF₃, -NH₂ or halogen, C₁-C₁₀-alkyl, -O-C₁-C₃-alkyl, -COOH, -COO-C₁-C₄-alkyl, or -CONH₂,
or
a group selected from among hydrogen, -CONH₂, -COOR⁷-OCON(R⁷)₂, -N(R⁷)₂, -NHCOR⁷-NHCON(R⁷)₂, -NO₂, CF₃, halogen, -O-C₁-C₆-alkyl-Q¹, -CONR⁷-C₁-C₁₀-alkyl-Q¹, -CONR⁷-C₁-C₁₀-alkenyl-Q¹, -CONR⁷-Q², halogen, OH, -SO₂R⁷, -SO₂N(R⁷)₂, -COR⁷, -COOR⁷, -N(R⁷)₂, -NHCOR⁷, -CONR⁷OC₁-C₁₀-alkyl-Q¹ and CONR⁷O-Q²,
or
adjacent groups R⁸ together denote a bridge of general formula a), b), c) or d), 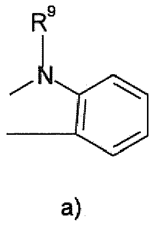 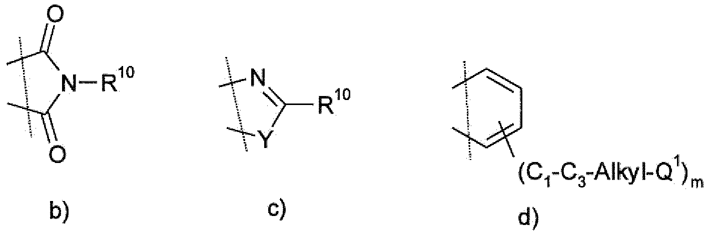 Y denotes O, S or NR¹¹,
m denotes 0, 1 or 2
R⁹ denotes C₁-C₆-alkyl
R¹⁰ denotes hydrogen or a group selected from among optionally substituted phenyl, pyridyl, pyrazinyl, pyrimidinyl, pyridazinyl, piperidinyl, piperazinyl, -C₁-C₃-alkyl-phenyl, -C₁-C₃-alkyl-pyridyl, -C₁-C₃-alkyl-pyrazinyl, -C₁-C₃-alkyl-pyrimidinyl and -C₁-C₃-alkyl-pyridazinyl,
wherein the above-mentioned alkyl, alkenyl and alkynyl groups may optionally be substituted by methyl, chlorine or fluorine, and
wherein the above-mentioned aryl groups may optionally be substituted by OH, NO₂, CN, OMe, -OCHF₂, -OCF₃, -NH₂, halogen, C₁-C₁₀-alkyl, -O-C₁-C₃-alkyl, -COOH, -COO-C₁-C₄-alkyl, or -CONH₂, and
wherein the above-mentioned heteroaryl groups may carry one or more of the substituents named bellow:
a) F, Cl, Br, OH, OMe, methyl, ethyl, CN, CONH₂, NH₂,
b) phenyl optionally substituted by OH, NO₂, CN, OMe, -OCHF₂, -OCF₃, -NH₂, halogen, C₁-C₁₀-alkyl, -O-C₁-C₃-alkyl, -COOH, -COO-C₁-C₄-alkyl, or -CONH₂,
c) heteroaryl optionally substituted by F, Cl, Br, OH, OMe, methyl, ethyl, CN, CONH₂ or NH₂,
d) or heteroaryl substituted by phenyl optionally substituted by OH, NO₂, CN, OMe, -OCHF₂, -OCF₃, -NH₂, halogen, C₁-C₁₀-alkyl, -O-C₁-C₃-alkyl, -COOH, -COO-C₁-C₄-alkyl, or -CONH₂,
R¹¹ denotes hydrogen or C¹-C⁴-alkyl
Q¹ denotes hydrogen, -NHCOR⁷, or a group selected from among an optionally substituted -NH-aryl, -NH-heteroaryl, aryl, heteroaryl, C₃-C₈-cycloalkyl and heterocycloalkyl group,
wherein the above-mentioned aryl groups may optionally be substituted by OH, NO₂, CN, OMe, -OCHF₂, -OCF₃, -NH₂, halogen, C₁-C₁₀-alkyl, -O-C₁-C₃-alkyl, -COOH, -COO-C₁-C₄-alkyl, or -CONH₂, and
wherein the above-mentioned heteroaryl groups may carry one or more of the substituents named below:
a) F, Cl, Br, OH, Me, methyl, ethyl, CN, CONH₂, NH₂,
b) phenyl optionally substituted by OH, NO₂, CN, OMe, -OCHF₂, -OCF₃, -NH₂, halogen, C₁-C₁₀-alkyl, -O-C₁-C₃-alkyl, -COOH, -COO-C₁-C₄-alkyl, or -CONH₂,
c) heteroaryl optionally substituted by F, Cl, Br, OH, OMe, methyl, ethyl, CN, CONH₂ or NH₂,
d) or heteroaryl substituted by phenyl optionally substituted by OH, NO₂, CN, OMe, -OCHF₂, -OCF₃, -NH₂**,** halogen, C₁-C₁₀-alkyl, -O-C₁-C₃-alkyl, -COOH, -COO-C₁-C₄-alkyl, or -CONH₂, and
wherein the above-mentioned cycloalkyl groups may optionally be substituted by OH, NO₂, CN, OMe, -OCHF₂, -OCF₃, -NH₂ or halogen, C₁-C₁₀-alkyl, -O-C₁-C₃-alkyl, -COOH, -COO-C₁-C₄₋alkyl, or -CONH₂,
Q² denotes hydrogen or a group selected from among an optionally substituted aryl, heteroaryl, C₃-C₈-heterocycloalkyl**,** C₃-C₈-cycloalkyl and C₁-C₄-alkyl-C₃-C₈-cycloalkyl group,
wherein the above-mentioned aryl groups may optionally be substituted by OH, NO₂, CN, OMe, -OCHF₂, -OCF₃, -NH₂, halogen, C₁-C₁₀-alkyl, -O-C₁-C₃-alkyl, -COOH, -COO-C₁-C₄-alkyl, or -CONH₂, and
wherein the above-mentioned heteroaryl groups may carry one or more of the substituents named below:
a) F, Cl, Br, OH, OMe, methyl, ethyl, CN, CONH₂, NH₂,
b) phenyl optionally substituted by OH, NO₂, CN, OMe, -OCHF₂, -OCF₃, -NH₂, halogen, C₁-C₁₀-alkyl, -O-C₁-C₃-alkyl, -COOH, -COO-C₁-C₄-alkyl, or -CONH₂,
c) heteroaryl optionally substituted by F, Cl, Br, OH, OMe, methyl, ethyl, CN, CONH₂ or NH₂,
d) or heteroaryl substituted by phenyl optionally substituted by OH, NO₂, CN, OMe, -OCHF₂, -OCF₃, -NH₂, halogen, C₁-C₁₀-alkyl, -O-C₁-C₃-alkyl, -COOH, -COO-C₁-C₄-alkyl, or -CONH₂, and
wherein the above-mentioned cycloalkyl groups may optionally be substituted by OH, NO₂, CN, OMe, -OCHF₂, -OCF₃, -NH₂ or halogen, C₁-C₁₀-alkyl, -O-C₁-C₃-alkyl, -COOH, -COO-C₁-C₄-alkyl, or -CONH₂,
and
n denotes 0, 1, 2, 3, 4 or 5.

6. Compounds according to one of claims 1 to 5,
wherein
Q¹, Q², n, R⁴ to R⁸ are as hereinbefore defined,
R¹, R² which may be identical or different denote hydrogen or a group selected from among methyl, ethyl, propyl, allyl and propargyl
or
R¹ and R² together represent cyclopropyl,
R³ is hydrogen, or denotes optionally substituted C₁-C₆-alkyl or optionally substituted C₃-C₁₂-cycloalkyl,
wherein the above-mentioned alkyl groups may optionally be substituted by methyl, chlorine or fluorine, and
wherein the above-mentioned cycloalkyl groups may optionally be substituted by OH, NO₂, CN, OMe, -OCHF₂, -OCF₃, -NH₂ or halogen, C₁-C₁₀-alkyl, -O-C₁-C₃-alkyl, -COOH, -COO-C₁-C₄-alkyl, or -CONH₂

7. Compounds according to claim 5 or 6,
wherein
Q¹, Q², n, R¹ to R⁴, R⁶ to R⁷ are as hereinbefore defined,
and
R⁸ which may be identical or different denote hydrogen or a group selected from among halogen, (C₁-C₂-alkyl)₂N-, CF₃, NH₂SO₂-, -CONH-C₆-C₁₄-aryl, -CONH-C₁-C₄-alkyl-C₆-C₁₄-aryl, -CONH-C₁-C₄-alkyl-C₆-C₁₄-heteroaryl, -CONH-C₃-C₈-cycloalkyl-heterocycloalkyl and -O-C₁-C₄-alkyl.

8. Compound of formula (I) according to one of claims 1 to 7 for use as a medicament.

9. Compound of formula (I) according to one of claims 1 to 7 for use as a medicament with an antiproliferative activity.

10. Use of a compound of formula (I) according to one of claims 1 to 7 for preparing a medicament for the treatment and/or prevention of diseases selected from among cancer, bacterial and viral infections, inflammatory and autoimmune diseases, chemotherapy-induced alopecia and mucositis, cardiovascular diseases, nephrological diseases, as well as chronic and acute neurodegenerative diseases.

11. Use of a compound of formula (I) according to one of claims 1 to 7 for preparing a medicament for inhibiting polo-like kinases.

12. Use according to claim 11, **characterised in that** the polo-like kinase is PLK1.

13. Use of a compound of formula (I) according to one of claims 1 to 7 for preparing a medicament for the treatment and/or prevention of tumoral diseases based on overexpression of the polo-like kinases.

14. Pharmaceutical preparations, containing as active substance one or more compounds of general formula (I) according to one of claims 1 to 7 optionally combined with conventional excipients and/or carriers.

## Revendications

1. Composés de formule générale (I) 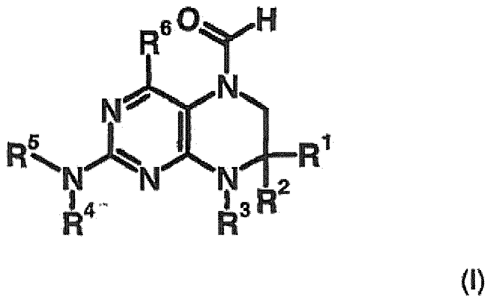 dans laquelle
R¹, R², identiques ou différents, représentent un radical choisi dans le groupe comprenant les groupes alkyle en C₁ à C₁₀ éventuellement substitué, alcényle en C₂ à C₁₀, alcinyle en C₂ à C₁₀, aryle, hétéroaryle, cycloalkyle en C₃ à C₈, hétérocycloalkyle en C₃ à C₈, -X-aryle, -X-hétéroaryle, -X-cycloalkyle, -X-hétérocycloalkyle, -NR⁷-aryle, -NR⁷-hétéroaryle, -NR⁷-cycloalkyle et -NR⁷-hétérocycloalkyle,
dans laquelle les radicaux alkyle, alcényle et alcinyle mentionnés précédemment peuvent être substitués éventuellement avec un groupe méthyle, un atome de chlore ou de fluor, et
dans laquelle les radicaux aryle mentionnés ci-dessus peuvent être substitués éventuellement par OH, NO₂, CN, OMe, -OCHF₂, -OCF₃, -NH₂, halogéno, alkyle en C₁ à C₁₀, -O-alkyle en C₁ à C₃, -COOH, -COO-alkyle en C₁ à C₄ ou -CONH₂, et
dans laquelle les radicaux hétéroaryle mentionnés ci-dessus peuvent porter un ou plusieurs des substituants mentionnés ci-après :
a) F, Cl, Br, NH, OMe, méthyle, éthyle, CN, CONH₂, -NH₂,
b) un groupe phényle éventuellement substitué par OH, NO₂, CN, OMe, -OCHF₂, -OCF₃, -NH₂, halogéno, alkyle en C₁ à C₁₀, -O-alkyle en C₁ à C₃, -COOH, -COO-alkyle en C₁ à C₄ ou -CONH₂,
c) un groupe hétéroaryle éventuellement substitué par F, Cl, Br, NH, OMe, méthyle, éthyle, CN, CONH₂ ou NH₂,
d) ou un groupe hétéroaryle substitué éventuellement par OH, NO₂, CN, OMe, -OCHF2, -OCF₃, -NH₂, halogéno, alkyle en C₁ à C₁₀, -O-alkyle en C₁ à C₃, -COOH, -COO-alkyle en C₁ à C₄ ou phényle substitué par -CONH₂, et
dans laquelle les radicaux cycloalkyle mentionnés ci-dessus peuvent être substitués éventuellement par OH, NO₂, CN, OMe, OCHF₂, -OCF₃, -NH₂ ou halogéno, alkyle en C₁ à C₁₀, -O-alkyle en C₁ à C₃, -COOH, -COO-alkyle en C₁ à C₄ ou -CONH₂, et
dans laquelle les radicaux hétérocycloalkyle mentionnés ci-dessus peuvent être substitués éventuellement par un groupe alkyle en C₁ à C₄,
ou
un radical choisi dans le groupe comprenant un atome d'hydrogène, d'halogène, COXR⁷, CON(R⁷)₂, COR⁷ et XR⁷, ou
R¹ et R² représentent conjointement un pont alkyle de 2 à 5 chaînons qui peut contenir 1 à 2 hétéroatomes,
R³ représente un atome d'hydrogène ou un radical choisi dans le groupe comprenant les groupes alkyle en C₁ à C₁₂ éventuellement substitué, alcényle en C₂ à C₁₂, alcinyle en C₂ à C₁₂, aryle, hétéroaryle, cycloalkyle en C₃ à C₁₂, cycloalcényle en C₃ à C₁₂, polycycloalkyle en C₇ à C₁₂, polycycloalcényle en C₇ à C₁₂ et spirocycloalkyle en C₅ à C₁₂,
les radicaux alkyle, alcényle et alcinyle mentionnés ci-dessus pouvant être substitués éventuellement par un groupe méthyle, un atome de chlore ou de fluor, et
les radicaux aryle mentionnés ci-dessus pouvant être substitués éventuellement par OH, NO₂, CN, OMe, -OCHF₂, -OCF₃, -NH₂, halogéno, alkyle en C₁ à C₁₀, -O-alkyle en C₁ à C₃, -COOH, -COO-alkyle en C₁ à C₄ ou -CONH₂, et
les radicaux hétéroaryle mentionnés ci-dessus pouvant porter un ou plusieurs des substituants suivants :
a) F, Cl, Br, OH, OMe, méthyle, éthyle, CN, CONH₂, NH₂,
b) un groupe phényle éventuellement substitué par OH, NO₂, CN, OMe, -OCHF₂, -OCF₃, -NH₂, halogéno, alkyle en C₁ à C₁₀, -O-alkyle en C₁ à C₃, -COOH, -COO-alkyle en C₁ à C₄ ou -CONH₂,
c) un groupe hétéroaryle éventuellement substitué par F, Cl, Br, OH, OMe, méthyle, éthyle, CN, -CONH2 ou NH₂,
d) ou un groupe hétéroaryle substitué éventuellement par OH, NO₂, CN, OMe, -OCHF₂, -OCF₃,-NH₂, halogéno, alkyle en C₁ à C₁₀, -O-alkyle en C₁ à C₃, -COOH, -COO-alkyle en C₁ à C₄ ou phényle substitué par -CONH₂, et
dans laquelle les radicaux cycloalkyle mentionnés ci-dessus peuvent être substitués éventuellement par OH, NO₂, CN, OMe, -OCHF2, -OCF₃, -NH₂ ou halogéno, alkyle en C₁ à C₁₀, -O-alkyle en C₁ à C₃, -COOH, -COO-alkyle en C₁ à C₄ ou -CONH₂,
ou
R¹ et R³ ou R² et R³ représentent conjointement un pont alkyle en C₃ à C₄ saturé ou insaturé qui peut contenir 1 à 2 hétéroatomes,
R⁴ représente un groupe aryle, benzyle ou hétéroaryle éventuellement substitué,
R⁵ représente un atome d'hydrogène, un groupe -CO-NH-alkyle en C₁ à C₄, -CO-alkyle en C₁ à C₄ ou -CO-X-alkyle en C₁ à C₄,
R⁶ représente un radical choisi dans le groupe comprenant un atome d'hydrogène, NH₂, XH, halogèno et un groupe alkyle en C₁ à C₃ éventuellement substitué par un ou plusieurs atomes d'halogène,
et
les R⁷ représentent, indépendamment les uns des autres, respectivement, un atome d'hydrogène ou un radical choisi dans le groupe comprenant un groupe alkyle en C₁ à C₄ éventuellement substitué, alcényle en C₂ à C₄, alcinyle en C₂ à C₄, benzyle et phényle,
les radicaux alkyle, alcényle et alcinyle mentionnés ci-dessus pouvant être substitués éventuellement par un groupe méthyle, un atome de chlore ou de fluor, et
les radicaux aryle mentionnés ci-dessus pouvant être substitués éventuellement par OH, NO₂, CN, OMe, -OCHF₂, -OCF₃, -NH₂, halogéno, alkyle en C₁ à C₁₀, -O-alkyle en C₁ à C₃, -COOH, -COO-alkyle en C₁ à C₄ ou -CONH₂,
X représente O ou S,
éventuellement sous la forme de leurs tautomères, leurs racémates, leurs énantiomères, leurs diastéréomères et leurs mélangés, et éventuellement leurs sels d'addition acides, solvates ou hydrates pharmacologiquement inoffensifs.

2. Composés selon la revendication 1,
dans lesquels
R¹ à R⁴ et R⁷ présentent la signification indiquée et
R⁵ et R⁶ représentent un atome d'hydrogène.

3. Composés selon la revendication 1 ou 2,
dans lesquels
R³ à R⁷ présentent la signification indiquée et
R¹, R², identiques ou différents, représentent un atome d'hydrogène ou un radical choisi dans le groupe consistant en un groupe alkyle en C₁ à C₆ éventuellement substitué, alcényle en C₂ à C₆ et alcinyle en C₂ à C₆,
les groupes alkyle, alcényle et alcinyle pouvant éventuellement être substitués par un groupe méthyle, un atome de chlore ou de fluor,
ou
R¹ et R² représentent conjointement un pont alkyle de 2 à 5 chaînons.

4. Composés selon l'une des revendications 1 à 3,
dans lesquels
R¹, R² et R⁴ à R⁷ présentent la signification indiquée et
les R³ représentent de façon identique un atome d'hydrogène ou un radical choisi dans le groupe comprenant un groupe alkyle en C₁ à C₁₂ éventuellement substitué, alcényle en C₂ à C₁₂, alcinyle en C₂ à C₁₂ et aryle en C₆ à C₁₄,
dans lesquels les radicaux alkyle, alcényle et alcinyle mentionnés ci-dessus peuvent être substitués éventuellement par un groupe méthyle, un atome de chlore ou de fluor, et
dans lesquels les radicaux aryle mentionnés ci-dessus peuvent être substitués éventuellement par OH, NO₂, CN, OMe, -OCHF₂, -OCF₃, -NH₂, halogéno, alkyle en C₁ à C₁₀, -O-alkyle en C₁ à C₃, -COOH, -COO-alkyle en C₁ à C₄ ou -CONH₂,
ou
un radical choisi dans le groupe consistant en les groupes cycloalkyle en C₃ à C₁₂, cycloalcényle en C₃ à C₁₂, polycycloalkyle en C₇ à C₁₂, polycycloalcényle en C₇ à C₁₂ et spirocycloalkyle en C₅ à C₁₂,
les radicaux cycloalkyle mentionnés ci-dessus pouvant être substitués éventuellement par OH, NO₂ CN, OMe, -OCHF₂, -OCF₃, -NH₂, halogéno, alkyle en C₁ à C₁₀, -O-alkyle en C₁ à C₃, -COOH, -COO-alkyle en C₁ à C₄, ou -CONH₂.

5. Composés selon l'une des revendications 1 à 4,
dans lesquels
R¹ à R³ et R⁵ à R⁷ présentent la signification indiquée et
R⁴ représente un radical de formule générale 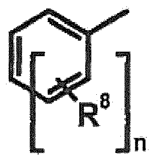 les R⁸ identiques ou différents, représentent un atome d'hydrogène ou un radical choisi dans le groupe comprenant les groupes alkyle en C₁ à C₆ éventuellement substitué, alcényle en C₂ à C₆, alcinyle en C₂ à C₆, -O-alkyle en C₁ à C₆, -O-alcényle en C₂ à C₆, -O-alcinyle en C₂ à C₆, hétérocycloalkyle, cycloalkyle en C₃ à C₆, aryle, hétéroaryle, -O-aryle, -O-hétéroaryle, -O-cycloalkyle et -O-hétérocycloalkyle,
les radicaux alkyle, alcényle et alcinyle mentionnés ci-dessus pouvant être substitués éventuellement par un groupe méthyle, un atome de chlore ou de fluor, et
les radicaux aryle mentionnés ci-dessus pouvant être substitués éventuellement par OH, NO₂, CN, OMe, OCHF₂, -OCF₃, -NH₂ ou halogéno, alkyle en C₁ à C₁₀, -O-alkyle en C₁ à C₃, -COOH, -COO-alkyle en C₁ à C₄ ou -CONH₂, et les radicaux hétéroaryle mentionnés ci-dessus pouvant porter un ou plusieurs des substituants mentionnés ci-après:
a) F, Cl, Br, OH, OMe, méthyle, éthyle, CN, CONH₂, NH₂,
b) un groupe phényle éventuellement substitué par OH, NO₂, CN, OMe, -OCHF₂, -OCF₃, -NH₂, halogéno, alkyle en C₁ à C₁₀, -O-alkyle en C₁ à C₃, -COOH, -COO-alkyle en C₁ à C₄ ou -CONH₂,
c) un groupe hétéroaryle éventuellement substitué par F, Cl, Br, NH, OMe, méthyle, éthyle, CN, CONH2 ou NH2,
d) ou un groupe hétéroaryle substitué éventuellement par OH, NO₂, CN, OMe, -OCHF₂, -OCF₃,-NH₂, halogéno, alkyle en C₁ à C₁₀, -O-alkyle en C₁ à C₃, -COOH, -COO-alkyle en C₁ à C₄ ou phényle substitué par -CONH₂, et
les radicaux cycloalkyle mentionnés ci-dessus pouvant être substitués éventuellement par OH, NO₂, CN, OMe, OCHF₂, -OCF₃, -NH₂ ou halogéno, alkyle en C₁ à C₁₀, -O-alkyle en C₁ à C₃, -COOH, -COO-alkyle en C₁ à C₄ ou -CONH₂,
ou
un radical choisi dans le groupe comprenant und atome d'hydrogène, -CONH₂, -COOR⁷, -OCON(R⁷)₂, -N(R⁷)₂, -NHCOR⁷, -NHCON(R⁷)₂, -NO₂, CF₃, halogène, -O-alkyl en C₁ à C₆-Q¹, -CONR⁷-alkyle en C₁ à C₁₀-Q¹, -CONR⁷-alcényle en C₁ à C₁₀-Q1, -CONR⁷-Q2, halogéno, OH, -SO₂R⁷, -SO₂N(R⁷)₂, -COR⁷, -COOR⁷, -N(R⁷)₂, -NHCOR⁷, -CONR⁷OC-alkyle en C₁ à C₁₀-Q¹ et CONR⁷O-Q₂,
ou
des radicaux R⁸ voisines représentent conjointement un pont de formule générale a), b), c) ou d), 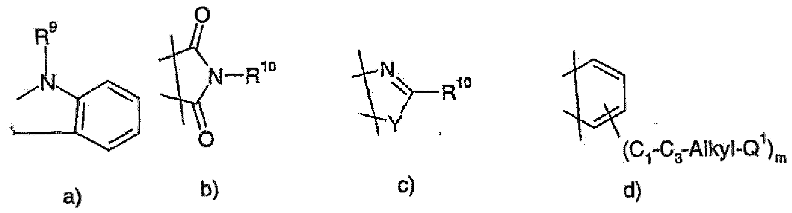 Y représente O, S ou NR¹¹,
m représente 0, 1 ou 2,
R⁹ représente und groupe alkyle en C₁ à C₆,
R¹⁰ représente un atome d'hydrogène ou un radical choisi dans le groupe comprenant les groupes phényle éventuellement substitué, pyridyle, pyrazinyle, pyrimidinyle, pyridazinyle, pipéridinyle, pipérazinyle, alkyle en C₁ à C₃-phényle, alkyle en C₁ à C₃-pyridyle, alkyle en C₁ à C₃-pyrazinyle, alkyle en C₁ à C₃-pyrimidinyle et alkyle en C₁ à C₃-pyridazinyle,
les radicaux alkyle, alcényle et alcinyle mentionnés ci-dessus pouvant être substitués éventuellement par un groupe méthyle, un atome de chlore ou de fluor, et
les radicaux acyle mentionnés ci-dessus pouvant être substitués éventuellement par OH, NO₂, CN, OMe, OCHF₂, -OCF₃, -NH₂, halogène, alkyl en C₁ à C₁₀, -O-alkyle en C₁ à C₃, -CONH, -COO-alkyle en C₁ à C₄ ou -CONH₂, et
les radicaux hétéroaryle mentionnés ci-dessus pouvant porter un ou plusieurs des substituants mentionnés ci-après :
a) F, Cl, Br, OH, OMe, méthyle, éthyle, CN, CONH₂, NH₂,
b) un groupe phényle éventuellement substitué pair OH, NO₂, CN, OMe, -OCHF₂, -OCF₃, -NH₂, halogéno, alkyle en C₁ à C₁₀, -O-alkyle en C₁ à C₃, -CONH, -COO-alkyle en C₁ à C₄ ou -CONH₂,
c) un groupe hétéroaryle éventuellement substitué par F, Cl, Br, OH, OMe, méthyle, éthyle, CN, CONH₂ ou NH₂,
d) ou un groupe hétéroaryle substitué éventuellement par OH, NO₂, CN, OMe, -OCHF₂, -OCF₃, NH₂, halogène, alkyle en C₁ à C₁₀, -O-alkyle en C₁ à C₃, -COOH, -COO-alkyle en C₁ à C₄ ou phényle substitué par CONH₂, et
les radicaux cycloalkyle mentionnés ci-dessus pouvant être substitués éventuellement par OH, NO₂, CN, OMe, OCHF₂, -OCF₃, -NH₂ ou halogène, alkyle en C₁ à C₁₀, -O-alkyl en C₁ à C₃, -COOH, -COO-alkyle en C₁ à C₄ ou -CONH₂,
R¹¹ représente un atome d'hydrogène ou un groupe alkyle en C₁ à C₄,
Q¹ représente un atome d'hydrogène, -NHCOR⁷ ou un radical choisi dans le groupe comprenant un groupe -NH-aryle éventuellement substitué, -NH-hétéroaryle, aryle, hétéroaryle, cycloalkyle en C₃ à C₈ et hétérocycloalkyle,
les radicaux aryle mentionnées ci-dessus pouvant être substitués éventuellement par OH, NO₂, CN, OMe, OCHF₂, -OCF₃, -NH₂ ou halogène, alkyle en C₁ à C₁₀, -O-alkyl en C₁ à C₃, -COOH, -COO-alkyle en C₁ à C₄ ou -CONH₂, et les radicaux hétéroaryle mentionnés ci-dessus pouvant porter un ou plusieurs des substituants mentionnés ci-après :
a) F, Cl, Br, OH, OMe, méthyle, éthyle, CN, CONH₂, NH₂,
b) und groupe phényle éventuellement substitué par OH, NO₂, CN, OMe, -OCHF₂, -OCF₃, -NH₂, halogène, alkyle en C₁ à C₁₀, -O-alkyle en C₁ à C₃, -COOH, -COO-alkyle en C₁ à C₄ ou -CONH₂,
c) un groupe hétéroaryle éventuellement substitué par F, Cl, Br, OH, OMe, méthyle, éthyle, CN, CONH₂ ou NH₂,
d) ou un groupe hétéroaryle substitué éventuellement par OH, NO₂, CN, OMe, -OCHF₂, -OCF₃, -NH₂, halogéno, alkyle en C₁ à C₁₀, -O-alkyl en C₁ à C₃, -COOH, -COO-alkyle en C₁ à C₄ ou phényle substitué par -CONH₂, et
les radicaux cycloalkyle mentionnées ci-dessus pouvant être substitués éventuellement par OH, NO₂, CN, OMe, -OCHF₂, -OCF₃, -NH₂ ou halogène, alkyle en C₁ à C₁₀, -O-alkyl en C₁ à C₃, -COOH, -COO-alkyle en C₁ à C₄ ou -CONH₂,
Q² représente un atome d'hydrogène ou un radical choisi dans le groupe comprenant un groupe aryle éventuellement substitué, hétéroaryle, hétérocycloalkyle en C₃ à C₈, cycloalkyl en C₃ à C₈ et alkyle en C₁ à C₄-cycloalkyle en C₃ à C₈,
les radicaux aryle mentionnées ci-dessus pouvant être substitués éventuellement par OH, NO₂, CN, OMe, -OCHF2, -OCF₃, -NH₂ ou halogéno, alkyl en C₁ à C₁₀, -O-alkyle en C₁ à C₃, -COOH, -COO-alkyle en C₁ à C₄ ou -CONH₂, et les radicaux hétéroaryle mentionnés ci-dessus pouvant porter un ou plusieurs des substituants mentionnées ci-après :
a) F, Cl, Br, OH, OMe, méthyle, éthyle, CN, CONH₂, NH₂,
b) un groupe phényle éventuellement substitué par OH, NO₂, CN, OMe, -OCHF₂, -OCF₃, -NH₂, halogène, alkyle en C₁ à C₁₀, -O-alkyl en C₁ à C₃, -COOH, -COO-alkyle en C₁ à C₄ ou -CONH₂,
c) un groupe hétéroaryle éventuellement substitué par F, Cl, Br, OH, OMe, méthyle, éthyle, CN, CONH₂ ou NH₂,
d) ou un groupe hétéroaryle substitué éventuellement par OH, NO₂, CN, OMe, -OCHF₂, -OCF₃, NH₂, halogène, alkyle en C₁ à C₁₀, -O-alkyle en C₁ à C₃, -COOH, -COO-alkyle en C₁ à C₄ ou phényle substitué par -CONH₂, et
les radicaux cycloalkyl mentionnés ci-dessus pouvant être substitués éventuellement par OH, NO₂, CN, OMe, -OCHF₂, -OCF₃, -NH₂ ou halogène, alkyle en C₁ à C₁₀, -O-alkyle en C₁ à C₃, -COOH, -COO-alkyle en C₁ à C₄ ou -CONH₂,
et
n représente 0, 1, 2, 3, 4 ou 5.

6. Composés selon l'une des revendications 1 à 5,
dans lesquels
Q¹, Q², n, R⁴ à R⁸ présentant la signification indiquée,
R¹, R², identiques ou différents, représentent un atome d'hydrogène ou un radical choisi dans le groupe comprenant les groupes méthyle, éthyle, propyle, allyle et propargyle
ou
R¹ et R² représentent conjointement un groupe cyclopropyle,
les R³ identiques représentent un atome d'hydrogène ou un groupe alkyle en C₁ à C₆ éventuellement substitué ou un groupe cycloalkyl en C₃ à C₁₂ éventuellement substitué,
les radicaux alkyle mentionnés ci-dessus pouvant être éventuellement substitués par un groupe méthyle, un atome de chlore ou de fluor, et
les radicaux cycloalkyl mentionnés ci-dessus pouvant être substitués éventuellement par OH, NO₂, CN, OMe, -OCHF₂, -OCHF₃, -NH₂ ou halogène, alkyle en C₁ à C₁₀, -O-alkyle en C₁ à C₃, -COOH, -COO-alkyle en C₁ à C₄ ou -CONH₂.

7. Composés selon la revendication 5 ou 6,
dans lesquels
Q¹, Q², n, R¹ à R⁴, R⁶ à R⁷ présentent la lignification indiquée
et
les R⁸ identiques ou différents, représentent un atome d'hydrogène ou un radical choisi dans le groupe comprenant un atome d'halogène, un groupe (alkyle en C₁ à C₂)₂N-, CF₃, NH₂SO₂-, -CONH-alkyle en C₆ à C₁₄-aryle, CONH-alkyle en C₁ à C₄-aryle en C₆ à C₁₄, -CONH-alkyle en C₁ à C₄-hétéroaryle en C₆ à C₁₄, -CONH-cycloalkyle en C₃ à C₈-hétérocycloalkyle et -O-alkyl en C₁ à C₄.

8. Composé de formule (I) selon l'une des revendications 1 à 7, pour l'utilisation comme médicament.

9. Composé de formule (I) selon l'une des revendications 1 à 7, pour l'utilisation comme medicament ayant un effet antiprolifératif.

10. Utilisation d'un composé de formule (I) selon l'une des revendications 1 à 7, pour la production d'un médicament pour le traitement et/ou la prétention de maladies choisies dans le groupe comprenait le cancer, les infections bactériennes et virales, les maladies inflammatoires et auto-immunes, l'alopécie et la mucosite induites par des agents chimiothérapeutiques, les maladies cardiovasculaires, les maladies néphrologiques et les maladies neurodégénératives chroniques et aiguës.

11. Utilisation d'un composé de formule (I) selon l'une des revendications 1 à 7 pour la production d'un medicament pour l'inhibition des kinases de type Prolo.

12. Utilisation selon la revendication 11, **caractérisée en ce que** la kinase de type Prolo est PLK1.

13. Utilisation d'un composté de formule (I) selon l'une des revendications 1 à 7, pour la production d'un médicament pour le traitement et/ou la prétention de maladies tumorales fondées sur une surexpression des kinases de type Polio.

14. Préparation pharmaceutiques, contenant comme substance active, un ou plusieurs composées de formule générale (I) selon l'une des revendications 1 à 7, éventuellement en combinaison avec des adjuvantes et/ou véhicules habituels.
